# EUROPEAN PATENT APPLICATION

(11) **EP 4 043 572 A1**
(43) Date of publication of application: **17.08.2022**
(21) Application number: 21156553.6
(22) Date of filing: 11.02.2021
(51) Int. Cl.: C12N 15/52, C12N 9/04, C12N 9/10, C12N 9/88, C12N 15/77, C12P 19/04

(54) **GLYCOSYLTRANSFERASE DEFICIENT CORYNEBACTERIUM FOR THE PRODUCTION OF FUCOSYLLACTOSE**

(71) Applicant: GALAB Laboratories GmbH, 21029 Hamburg (DE)
(72) Inventor: Reddy, Siva Reddy Maram, 502 319 Telangana (IN); Kuballa, Jürgen, 21029 Hamburg (DE)
(74) Representative: Hertin und Partner Rechts- und Patentanwälte PartG mbB

(57) **Abstract**

The invention relates to a genetically modified *corynebacterium* for production of fucosyllactose, wherein the *corynebacterium* has been modified to express a permease for lactose import, GDP-D-mannose-4,6-dehydratase (GMD), GDP-L-fucose synthase (WcaG) and fucosyltransferase (FucT) from exogenous nucleic acid sequences, characterized in that the exogenous nucleic acid sequences encoding a permease for lactose import, GMD, WcaG and FucT are chromosomally integrated. Preferably, the *corynebacterium* additionally comprise chromosomally integrated exogenous nucleic acid sequences for expression of phosphomannomutase (ManB) and GTP-mannose-1-phosphate guanylyltransferase (ManC). In preferred embodiments, the *corynebacterium* is *corynebacterium glutamicum.* In embodiments, the *corynebacterium* of the invention can be defective for functional expression of one or more glycosyltransferases involved in *corynebacterial* cell wall biosynthesis.

## Description

The invention relates to a genetically modified *corynebacterium* for production of fucosyllactose, wherein the *corynebacterium* has been modified to express a permease for lactose import, GDP-D-mannose-4,6-dehydratase (GMD), GDP-L-fucose synthase (WcaG) and fucosyltransferase (FucT) from exogenous nucleic acid sequences, characterized in that the exogenous nucleic acid sequences encoding a permease for lactose import, GMD, WcaG and FucT are chromosomally integrated. Preferably, the *corynebacterium* additionally comprise chromosomally integrated exogenous nucleic acid sequences for expression of phosphomannomutase (ManB) and GTP-mannose-1-phosphate guanylyltransferase (ManC). In preferred embodiments, the *corynebacterium* is *corynebacterium glutamicum.* In embodiments, the *corynebacterium* of the invention can be defective for functional expression of one or more glycosyltransferases involved in *corynebacterial* cell wall biosynthesis.

### BACKGROUND OF THE INVENTION

Human breast milk contains more than 200 structurally distinct oligosaccharides, hereinafter referred to as Human milk oligosaccharides (HMOs), with typical concentration, ranges between 5 to 25 g/l. Their composition and concentration vary during lactation and further depend on the maternal genetics, diet, and health status. The presence of HMOs significantly mitigates acute infections in infants during/until the maturation of their immune system. Several HMOs used in clinical practice have proven to protect infants and adults from bacterial and viral infections. Additionally, some of the HMO constituents were reported to promote cognitive development and stimulate beneficial gut microbiota in infants. Hence, HMOs have gained increased attention in recent years as therapeutic agents and healthy supplements of human nutrition.

Fucosylated HMOs, in particular, α1,2-fucosyllactose (2FL) and α1,3-fucosyllactose (3FL) are dominant oligosaccharides in breast milk constituted in the range of 12-45% and 0.5-8% respectively. 2'-FL (Fucα1-2[Galβ1-4]Glc) consists of L-fucose linked by an α1-2 bond to the galactose residue in lactose, and its isomer 3-FL (Fucα1-3Galβ1-4Glc) consists of L-fucose linked by an α1-3 bond to the glucose moiety in lactose. 2FL promotes early incidence of Bifidobacteria in gut microbiota and provides protection against diarrheal diseases caused by commensals in infants. it is known that about 20% of European and American women cannot synthesize 2FL due to the genetic mutation of fucosyltransferase genes. And in women who can produce 2FL, its concentration in milk decreases over the period of lactation. Hence, 2FL attracted much interest as a functional food ingredient in infant formula. For these reasons, there is a need to produce 2FL.

Fucosylated HMOs are either absent or present at a 100-to-1000-fold lower concentration in the milk of farm animals. The composition and variety of oligosaccharides from farm animals significantly differ from HMOs, besides extraction from animal milk or whey is not a commercially viable option for largescale production. Chemical synthesis is challenging due to multistep synthesis and accompanied by the cons of employing expensive substrates, low stereoselectivity, production yield, and the use of toxic organic solvents. Biocatalytic synthesis is of limited applicability to scale-up due to the use of expensive substrates and unstable and low expression of fucosyltransferases. 2FL production using recombinant microorganisms subdue the drawbacks of chemical and enzymatic synthesis with the added advantage of scale-up and industrial production from inexpensive substrates.

Most conventional methods for producing 2'-fucosyllactose using microorganisms were produced using recombinant *Escherichia coli.* However, most *Escherichia coli* used for experimentation are predominantly known to be harmful to customers although they are not pathogens. Since an ingredient for the cell membrane of Escherichia coli may serve as endotoxin, high isolation, and purification (downstream processing) costs are involved in the production of 2'-fucosyllactose from *Escherichia coli.* Accordingly, there is a difficulty in using *Escherichia coli* as a host cell that produces fucosyllactose as food and medicinal materials ingredients.

To avoid the drawback of using the (potential) human pathogen *E. coli* for fucosyllactose production, US20200048640A1 uses recombinant *Corynebacterium glutamicum* for producing fucosyllactose. However, the bacteria described therein contain the transgenes encoding for enzymes of the production pathway of fucosyllactose on plasmids that can easily be lost without constant antibiotic selection. Therefore, producing fucosyllactose employing this recombinant bacterium requires extensive downstream processing steps to purify 2FL. This is an important drawback of the described method since the produced 2FL can comprise antibiotics, which is a huge disadvantage for the intended subsequent use in baby food.

Because of the drawbacks of known methods and means for producing fucosyllactose, there remains a significant need in the art to provide additional means and methods for industrial production of fucosyllactose in a non-pathogenic and generally regarded as safe (GRAS) microorganism while avoiding the use of antibiotics during the production method.

Furthermore, the use of *Corynebacterium glutamicum* for producing biomolecules has been reported to be disadvantageous due to the strong tendency of the bacteria to produce foam during fermentation. In fact, agitation and aeration of *Corynebacterium glutamicum* cultures in a stirred tank bioreactor always cause foaming, and excess foaming forces the broth out of the bioreactor and contaminates the system. Accordingly, laborious optimization experiments must be performed to identify optimal aeration conditions that must be maintained to minimize foaming and maximize the specific production rate.

Accordingly, there is need in the art for means and methods that reduce foaming of *Corynebacterium glutamicum* in the context of the production of biomolecules such as fucosyllactose by these bacteria.

### SUMMARY OF THE INVENTION

Considering the prior art, the technical problem underlying the present invention is to provide alternative and/or improved means to produce fucosyllactose in transgenic bacteria. Furthermore, the provision of means and methods employing *Corynebacterium glutamicum* for producing biomolecules that reduce foam production by the bacterial cultures and therefore facilitate culture of these bacteria for industrial applications, ideally with increase production yields, are desired.

The problems underlying the present invention are solved by the features of the independent claims. Preferred embodiments of the present invention are provided by the dependent claims.

### Aspects of the invention relating to genetically modified corynebacterium with chromosomally integrated transgenes required for production of fucosyllactose

The invention therefore relates to genetically modified (or engineered) *corynebacterium* for production of fucosyllactose, wherein the *corynebacterium* has been modified to express a permease for lactose import, GDP-D-mannose-4,6-dehydratase (GMD), GDP-L-fucose synthase (WcaG) and fucosyltransferase (FucT) from exogenous nucleic acid sequences,
**characterized in that** the exogenous nucleic acid sequences encoding a permease for lactose import, GMD, WcaG and FucT are chromosomally integrated.

The development of the genetically modified *corynebacterium* of the invention is highly advantageous in comparison to the state of the art. The bacteria of the invention enable highly effective production of fucosyllactose in industrial scale, for example for subsequent nutritional applications, in the absence of any antibiotics. Furthermore, the *corynebacteria* and in particular the preferred *corynebacterium glutamicum* is not pathogenic in humans and has been considered as a generally recognized as safe (GRAS) host for the industrial production of biomolecules, such as in particular L-glutamate and L-lysine.

Importantly, most known processes for producing fucosyllactose, in GRAS hosts, have used the expression of plasmid-borne genes to express heterologous enzymes of the pathway in the host. Replicating plasmids are mostly useful for cloning and overexpression of recombinant genes, for the maximum overproduction of a given protein or for short-term lab scale synthesis of a product. However, for the in vivo synthesis of a natural product such as fucosyllactose in a bacterial host, where multiple genes must be expressed, the use of expression plasmids has several disadvantages. Different plasmids exhibit variable copy numbers based on the origin of replication, and consequently, differential expression of genes of the multi-enzymatic pathway results in flux imbalances thence poor product yields. Additionally, multicopy plasmids are segregationally unstable and detrimental to industrial-scale manufacturing processes. Particularly during high cell density fermentation and continuous fermentation conditions, there is the incidental loss of selective pressure due to reduced antibiotic activity because of degradation of antibiotics by the cell and culture dilution respectively. Auxotrophic strains although can maintain plasmids bearing necessary genes without antibiotics and selective pressure are also susceptible to plasmid loss due to leakage of the complementing product from the plasmid-bearing cell. Thus, the expression of the genes often fluctuates in the cell population, which results in population variation.

Moreover, the metabolic burden of plasmid replication and the strong overexpression of certain genes can lead to reduced growth rates and increased demand for energy and metabolites for the additional pathways. Structural instability of plasmids or transposon insertion in to expressing genes or gene loss due to recombination between multiple vectors lead to the takeover of culture by plasmids with inactivated genes.

Most importantly, in processes aimed at human nutrition, the use of antibiotics is either legally forbidden, not indicated or disadvantageous for safety reasons, or undesired for cost reasons. In addition, the removal of antibiotics in downstream processing is elaborate and expensive.

To avoid these disadvantages, stable insertion of recombinant genes into the chromosome of the *corynebacteria* of the invention is performed. The chromosomal integration allows the stable maintenance of gene expression cassettes without the use of selection marker in strains.

However, while methods for chromosomal integration of expression cassettes are well established in E. coli such techniques have been proven difficult for performance in other bacteria and in particular in corynebacteria and *corynebacterium glutamicum.*

However, as shown in the examples below, it was surprisingly possible to efficiently construct genetically modified *corynebacteria* that comprise multiple expression cassettes (also called gene cassettes) for all required and advantageous genes of the fucosyllactose synthesis pathway. This was unexpected since the present invention required the integration of multiple expression cassettes into the bacterial chromosome. However, it is known that for *corynebacterium glutamicum* genome modification by the integration of expression cassettes is laborious, time-consuming, and inefficient due to the rare event of genomic integration of the plasmid accompanied with screening several colonies after the second recombination to identify the desired recombination event. This process often becomes problematic when integrating multiple DNA fragments, especially if the expression cassettes have homologous sequences. The homologous sequences in different expression cassettes are problematic in generating strains because the cassettes may result in illegitimate recombination with each other. Hence it is a limitation to integrate multiple copies of the same gene in the chromosome which is an important prerequisite to regulate the expression of multiple genes in the pathway.

In the context of the present invention, the difficulty of integrating multiple DNA fragments and avoided illegitimate recombination could be overcome by using expression cassettes with heterologous sequences encoding the respective gene functions from different organisms and by minimizing multiple usage of promotor and terminator sequences. Furthermore, insertion sites are chosen in between essential genes on the chromosomes to subdue recombination between expression cassettes with homologous sequences.

Accordingly, in embodiments, the chromosomally integrated exogenous nucleic acid sequences encoding a permease for lactose import, GMD, WcaG and FucT comprise multiple expression cassettes which are integrated in different chromosomal intergenic regions. Preferably, the integration/insertion sites of the cassettes are located in different intergenic regions between different essential genes of the bacteria.

In embodiments, the genetically modified bacteria of the invention comprise 3, 4, 5, 6, 7, 8, 9 or 10 expression cassettes. Preferably, the genetically modified bacteria of the invention comprise at least 5, 6 or 8 expression cassettes.

In embodiments, each expression cassette of the transgenic bacterium of the invention has a different promoter sequence. In preferred embodiments, no more than two of the expression cassettes of the transgenic bacteria comprise the same promoter. In embodiments, no more than three of the expression cassettes of the transgenic bacteria comprise the same promoter. In embodiments where two or more expression cassettes comprise the same promoter, it is preferred that the promoter present in two or more of the expression cassettes is the Tuf and/or Tac promoter.

In embodiments, each expression cassette of the transgenic bacterium of the invention has a different terminator sequence. In preferred embodiments, no more than two of the expression cassettes of the transgenic bacteria comprise the same terminator. In embodiments, no more than three of the expression cassettes of the transgenic bacteria comprise the same terminator.

In embodiments, the expression cassettes comprise one or more heterologous promoters. In embodiments, the expression cassettes comprise one or more corynebacterial promoters. In embodiments, the expression cassettes comprise one or more heterologous promoters and one or more corynebacterial promoters.

In embodiments comprising corynebacterial promoters, such as Tuf and/or GlyA promoters from corynebacterium, the expression cassettes comprising corynebacterial promoters are integrated at intergenic sites spanning essential genes.

Additionally, there is a need for high-level expression of selective pathway genes to improve flux towards fucosyllactose production from glycolytic intermediate Fructose - 6 - phosphate without impairing cell growth, which needs testing both constitutive and regulated promotors. Gene dosage and promotor strength are important considerations during strain construction process.

In the context of the present invention, high expression of all transgenes of the fucosyllactose production pathways could be ensured by using a specific selection of inducible promoters for expression of regulatory genes in the pathway especially GMD and ManB. For example, in the preferred example MB02 described herein, ManB_ManC and GMD_WcaG operons are subjected to inducible expression. It can also be preferred that FucT is under inducible expression, as described herein for HpFucT in the base strain MB002. To achieve inducible expression of the pathway genes, a gene cassette of Lactose repressor gene Lacl driven from a Laclq promotor is introduced in the chromosome additionally.

Further improvement in 2FL production can be attained with constitutive expression of additional genes. In embodiments, a WcaG-GMD operon is under the control of a constitutive promotor, such as a Tuf promoter, with Lac Operator to effectuate IPTG inducible expression to avoid growth impairment during early and log phase.

In addition, there is evidence from the studies prokaryotes and eukaryotes that chromosomal location can substantially affect heterologous gene expression levels (1,2). Chromosomal position and spatial organization of gene cassettes is important to maintain gene dosage and expression. These positional effects are poorly understood in Corynebacterium and need to be tested to mitigate potential expression silencing of integrated genes cassettes (see for example Bryant, J. A. et al. "Chromosome position effects on gene expression in Escherichia coli K-12." Nucleic Acids Res. 42, 11383-11392 (2014); Wilson C. et al. "Position effects on eukaryotic gene expression." Annu. Rev. Cell Biol. 1990;6:679-714).

Appropriate selection of chromosome location is important to maintain reliable gene dosage. Mobile genetic elements such as transposase genes are detrimental to heterologous protein expression (Choi, Jae Woong, et al. "Enhanced Production of Recombinant Proteins with Corynebacterium Glutamicum by Deletion of Insertion Sequences (IS Elements)." Microbial Cell Factories, vol. 14, no. 1, 2015, pp. 207-207).

In preferred embodiments of the invention, suitable chromosomal integration sites for the exogenous nucleic acid sequences (transgenes/expression cassettes) were selected to integrate gene cassettes with concomitant deletion of transposase gene. Reproducible gene expression from selected sites is determined based on relative expression of an integrated reporter gene cassette, such as preferably the *E*. *coli* galactosidase (LacZ) gene under the control of constitutive LacuV5 promotor. Sites with high LacZ expression levels are selected further to integrate 2FL pathway gene cassettes. Accordingly, in embodiments the integration sites of the exogenous nucleic acid sequences of genetically modified corynebacterium have been selected based on suitability for exogenous gene expression as assessed using a reporter gene expression cassette, and preferably also suitability for integration of 2FL pathway gene cassettes.

Preferred integration sites for exogenous nucleic acid sequences of the invention have been disclosed in the examples below. In the context of the present invention, preferred integration sites for expression cassettes comprise integrations leading to deletion of following transposase genes: Δ*cgp_2725,* Δ*cgp_1213,* Δ*cgp_2854,* Δ*cgp_3151,* Δcgp_1178, Δ*cgp_1782,* Δ*cgp_2600.*

As used herein, the term genetically modified *corynebacterium* and genetically engineered *corynebacterium* are used interchangeably.

Besides the provision of exogenous fucosyltransferase (FucT), *Corynebacterium* requires incorporation of GDP-D-mannose-4,6-dehydratase (Gmd), GDP-L-fucose synthase (this enzyme is also called "GDP-4-keto-6-deoxy-D-mannose-3,5-epimerase-4-reductase" and is also simply referred to as "WcaG"), and a permease for lactose import, such as lactose permease (LacY).

Importantly, in contrast to approaches for fucosyllactose production using *E*. *coli,* where *E*. *coli* has genes encoding Gmd, WcaG, and lactose permease (LacY), *Corynebacterium glutamicum* has no genes encoding these enzymes, so provision of external coding nucleic acid sequences for expression of these genes is required.

In embodiments, genes encoding α-1,2-fucosyltransferase are derived from *Helicobacter pylori,* and genes encoding Gmd, WcaG and lactose permease (LacY) are derived from *Escherichia coli.*

In embodiments, the *corynebacterium* additionally comprise chromosomally integrated exogenous nucleic acid sequences for expression of phosphomannomutase (ManB) and GTP-mannose-1-phosphate guanylyltransferase (ManC).

*Corynebacterium glutamicum* possesses genes encoding phosphomannomutase (ManB) and GTP-mannose-1-phosphate guanylyltransferase (ManC) and can thus express the same. Therefore, it may not be absolutely required that these genes are further expressed from exogenous nucleic acid sequence. However, increased expression of these enzymes is advantageous for mass-production of fucosyllactose and for this reason, the present invention preferably comprises chromosomally integrated exogenous nucleic acid sequences for expression of ManB and ManC.

In preferred embodiments, the *corynebacterium* additionally comprise a chromosomally integrated exogenous nucleic acid sequence, such as an expression cassette, for expression of a lactose repressor. A lactose repressor (or lac repressor; encoded by a lactose repressor gene) is a DNA-binding protein that inhibits the expression of transgenes of the invention present in an expression cassette comprising the lac operon or parts thereof in the absence of lactose or IPTG. Corresponding inducible genetic systems for inducible transgene expression are known in the art. In the context of the invention, Lac repressor controlled inducible expression of heterologous genes can comprise the use of Lac/IPTG inducible promotors such as Lac, Tac, Trc etc. In embodiments, synthetic chemical IPTG can be also used for induction of respective genes.

In the context of the present invention, lactose is provided for the synthesis of FL by the transgenic bacteria of the invention. It is advantageous to include a transgenically expressed lac repressor in the bacteria of the invention. Suitable lac repressors of the invention include Lacl gene from *E. coli.*

In embodiments, expression cassettes of the invention comprise a promoter and a coding sequence. In embodiments, expression cassettes of the invention comprise a promoter, an operator and a coding sequence. In embodiments, expression cassettes of the invention comprise a promoter, an operator, a terminator sequence and a coding sequence. In embodiments, expression cassettes of the invention comprise a promoter, an operator, a terminator sequence and/or a coding sequence. In embodiments, such expression cassettes may comprise more than one coding sequence.

Preferred promoters to be used in the context of the invention comprise Tac promoter, Tuf promoter, GlyA promoter, Lac promoter and Trc promoter. Preferred operators of the invention comprise the lac operator. Preferred terminators comprise T7 terminator and rrnB terminator.

Preferred promoters to be used for the transgenes of the invention have been described in the examples below. The individual expression cassettes described therein, including cassettes for single genes or cassettes comprising more than one gene, such as the operons described therein, can be used independently in the context of the invention have not to be used in the exact combination disclosed in the examples below, as is understood by the skilled person.

In embodiments of the genetically modified corynebacterium for production of fucosyllactose, ManC and ManB are encoded by a continuous exogenous nucleic acid sequence comprising coding sequences for ManC and ManB within a single operon. Such an operon may be controlled by a single promoter, such as a Tac promoter.

In embodiments, ManC and ManB are encoded by separate (non-continuous) exogenous nucleic acid sequence and preferably comprise independent regulatory sequences. The respective coding sequences can be integrated at independent locations within the *corynebacterium* chromosome.

As disclosed herein, the *corynebacteria* of the invention can comprise each transgene separately, e.g. in a separate exogenous nucleic acid sequence preferably with independent regulatory sequences. In alternative embodiments, two or more of the transgenes of the *corynebacteria* of the invention can be comprised/encoded by a continuous exogenous nucleic acid sequence comprising coding sequences of the two or more transgenes within a single operon.

Each of the transgenes of the *corynebacterium* of the invention can be present in more than one copy, such as 2, 3, 4, 5, 6, 7, 8, 9 or 10 copies, wherein the number of copies of one transgene can be independent of the number of copies of the other transgenes. Therein, the copies of one transgene can be of the same (identical coding sequences of the respective transgene) or different variants of the transgene, such as transgenes of different origins. For example, in case of FucT, the bacteria of the invention can comprise two exogenous nucleic acid sequences encoding FucT (two copies), wherein one copy can be, for example, a FucT encoding sequence from *Helicobacter pylori,* and the other copy can be, for example, a FucT encoding sequence from *Helicobacter mustelae.* The same holds true for each of the different transgenes of the *corynebacterium* of the invention.

In preferred embodiments, the *corynebacterium* is *corynebacterium glutamicum.*

*Corynebacterium glutamicum* is a frequently used and important microorganisms in white biotechnology, due to its classification as a GRAS host.

In embodiments, the genetically modified *corynebacterium* of the invention exports fucosyllactose.

In embodiments of the invention, the genetically modified *corynebacterium* is engineered to export fucosyllactose into the medium when grown at appropriate culture conditions. It is understood that export of FL relates to any kind of process, active or passive, that leads to FL being present outside the bacteria in the culture medium.

In embodiments, the FucT comprised by the genetically engineered *corynebacterium* of the invention is alpha-1,2-FucT. In further embodiments, the FucT comprises alpha-1,2-FucT.

To produce 2'-FL, the use of a suitable alpha-1,2-FucT is preferred. *Corynebacteria* comprising alpha-1,2-FucT from *Helicobacter pylori* are particularly preferred. Alternatively, or in addition, alpha 1,2FucT WbgL from Ecoli 0126 or *Thermosynechoccus elongatus* may be used. Alternatively, or in addition, the alpha-1,2-FucT from *Helicobacter mustelae* may be used.

In embodiments, the permease for lactose import comprised by the genetically engineered *corynebacterium* of the invention is a lactose permease (LacY).

In embodiments, the *corynebacterium* comprises at least one exogenous nucleic acid sequences encoding FucT, wherein a FucT coding sequence is from *Helicobacter pylori.*

In embodiments, the *corynebacterium* comprises at least one exogenous nucleic acid sequences encoding FucT, wherein expression of a FucT coding sequence is controlled by a Trc promoter.

In embodiments, the *corynebacterium* comprises at least one exogenous nucleic acid sequences encoding FucT, wherein a FucT coding sequence is from *Helicobacter pylori* and expression of a FucT coding sequence is controlled by a Trc promoter.

In embodiments, the *corynebacterium* comprises at least one exogenous nucleic acid sequences encoding FucT, wherein a FucT coding sequence is codon optimized from *Helicobacter mustelae.*

In embodiments, the *corynebacterium* comprises at least one exogenous nucleic acid sequences encoding FucT, wherein expression of a FucT coding sequence is controlled by a Tuf promoter.

In embodiments, the *corynebacterium* comprises at least one exogenous nucleic acid sequences encoding FucT, wherein a FucT coding sequence is codon optimized from *Helicobacter mustelae* and expression of a FucT coding sequence is controlled by a Tuf promoter.

In embodiments, the *corynebacterium* comprises at least one exogenous nucleic acid sequences encoding FucT, wherein a FucT coding sequence is from *Helicobacter pylori* and expression of a FucT coding sequence is controlled by a Tuf promoter.

In embodiments, the *corynebacterium* comprises at least one exogenous nucleic acid sequences encoding FucT, wherein a FucT coding sequence is codon optimized from *Helicobacter mustelae* and expression of a FucT coding sequence is controlled by a Trc promoter.

In embodiments, the *corynebacterium* comprises at least two exogenous nucleic acid sequences encoding FucT.

In embodiments, the *corynebacterium* comprises at least two exogenous nucleic acid sequences encoding FucT, wherein preferably
i. a first FucT coding sequence is from *Helicobacter pylori* and/or expression of a first FucT coding sequence is controlled by a Trc promoter, and
ii. a second FucT coding sequence is codon optimized from *Helicobacter mustelae* and/or expression of a second FucT coding sequence is controlled by a Tuf promoter.

In embodiments of the *corynebacterium* of the invention, the *corynebacterium* comprises at least two exogenous nucleic acid sequences encoding FucT.

In embodiments, the *corynebacterium* of the invention comprises a FucT from *Helicobacter pylori.*

In embodiments, the *corynebacterium* of the invention comprises a FucT coding sequence controlled by a Trc promoter.

In embodiments, the *corynebacterium* of the invention comprises a FucT from *Helicobacter mustelae.*

In embodiments, the *corynebacterium* of the invention comprises a FucT coding sequence controlled by a Tuf promoter.

In embodiments, the FucT comprised by the *corynebacterium* of the invention comprises a FucT from *Helicobacter pylori* and a FucT from *Helicobacter mustelae.*

In preferred embodiments of the genetically modified *corynebacterium* of the invention, WcaG and GMD are encoded by a continuous exogenous nucleic acid sequence comprising coding sequences for WcaG and GMD within a single operon.

In embodiments, WcaG and GMD are encoded by separate (non-continuous) exogenous nucleic acid sequence and preferably comprise independent regulatory sequences. The respective coding sequences can be integrated at independent locations within the *corynebacterium* chromosome.

In embodiments, the *corynebacterium* of the invention comprises at least one exogenous nucleic acid sequence encoding WcaG and GMD are encoded as a single fusion gene expressing both the activities of GMD and WcaG.

In embodiments, the coding sequences for WcaG and GMD are from *Escherichia coli.*

In embodiments, the genetically modified *corynebacterium* of the invention comprises at least one exogenous nucleic acid sequences encoding WcaG and GMD in a continuous exogenous nucleic acid sequence within a single operon. In embodiments, a WcaG and GMD encoding operon is controlled by a Tac promoter. In embodiments, the coding sequences of WcaG and/or GMD are from *Escherichia coli.* In embodiments, a WcaG and GMD encoding operon is controlled by a Tuf promoter. In embodiments, the coding sequences of WcaG and/or GMD are from *Bacteroides fragilis.*

In embodiments, the genetically modified *corynebacterium* of the invention comprises at least two exogenous nucleic acid sequences encoding WcaG and GMD in a continuous exogenous nucleic acid sequence within a single operon, wherein preferably
i. a first WcaG and GMD encoding operon is controlled by a Tac promoter and/or the coding sequences are from *Escherichia coli,* and
ii. a second WcaG and GMD encoding operon is controlled by a Tuf promoter and/or the coding sequences are from *Bacteroides fragilis.*

In embodiments, the genetically modified *corynebacterium* of the invention comprises atleast one exogenous nucleic acid sequence encoding a permease for lactose import. In embodiments, expression of a permease for lactose import coding sequence is controlled by a glyA promoter. In embodiments, a permease for lactose import is LacY from *Lactobacillus delbrueckii.* In embodiments, expression of a permease for lactose import coding sequence is controlled by a constitutive Lac promoter. In embodiments, a permease for lactose import is LacY from *Escherichia coli.*

In embodiments, the genetically modified *corynebacterium* of the invention comprises at least two exogenous nucleic acid sequences encoding a permease for lactose import, wherein preferably
i. expression of a first permease for lactose import coding sequence is controlled by a glyA promoter and/or a first permease for lactose import coding sequence is LacY from *Lactobacillus delbrueckii,* and
ii. expression of a second permease for lactose import coding sequence is controlled by a constitutive Lac promoter and/or is LacY from *Escherichia coli.*

In preferred embodiments, the *corynebacterium* is *corynebacterium glutamicum* and comprises
- at least two exogenous nucleic acid sequences encoding FucT,
- at least two exogenous nucleic acid sequences encoding WcaG
- at least two exogenous nucleic acid sequences encoding GMD, and
- at least two exogenous nucleic acid sequences encoding LacY.

In such embodiments, exogenous nucleic acid sequences preferably comprise coding sequences for WcaG and GMD in a continuous nucleic acid sequence within a single operon. In preferred embodiments, *corynebacteria* of the invention comprise two (or more) such operons with coding sequence for WcaG and GMD.

In preferred embodiments, the corynebacterium is *corynebacterium glutamicum* and comprises
- at least two exogenous nucleic acid sequences encoding FucT,
- at least two exogenous nucleic acid sequences encoding WcaG and GMD in a continuous exogenous nucleic acid sequence within a single operon, and
- at least two exogenous nucleic acid sequences encoding LacY.

In embodiments, the genetically modified *corynebacterium* of the invention is defective for functional expression of one or more glycosyltransferases involved in *corynebacterial* cell wall biosynthesis.

Surprisingly, it was found out that deficiency in glycosyltransferases involved in *corynebacterial* cell wall biosynthesis does not affect the efficiency of intracellular metabolic pathways a in particular not of an introduced fucosyllactose biosynthesis pathway.

Importantly, however, such a functional deficiency that can be caused by inactivation of one or more of such glycosyltransferases reduce the foam production by *corynebacterial* cultures during production processes and therefore make such processes much easier to handle. Furthermore, it is possible to scale up the volumes of the bacterial cultures, since no additional space in the culture vessels/containers has be reserved for foam that is produced during the culture process.

Unexpectedly, the reduction of foam does not only lead to such practical advantages for the culture and production process, but it turned out that deficiency for one or more glycosyltransferases involved in *corynebacterial* cell wall biosynthesis also allows a more efficient production and purification of fucosyllactose from the bacterial culture. Therefore, besides and independent from the possibility of culturing larger volumes, it is also possible to purify higher amounts of fucosyllactose from identical culture volumes in comparison to producing *corynebacteria* that have are not defective for one or more glycosyltransferases involved in *corynebacterial* cell wall biosynthesis.

Therein, the one or more glycosyltransferases are preferably selected from the group comprising or consisting of cgp_3313 (MrcB; GT51), cgp_0336 (PonA; GT51); cgp_3166 (GT4); cgp_2400 (GT4); cgp_1876 (GT4); cgp_1268 (GlgA; GT4); cgp_0554 (GT4); cgp_3191 (GlfT; GT2); cgp_1672 (PpmC; GT2); cgp_1180 (GT2); cgp_0848 (WbbL; GT2); cgp_0730 (GT2); cgp_0396 (GT2); cgp_0394 (GT2); cgp_0246 (GT2); cgp_0163 (GT2); cgp_2393 (GT87,GT87); cgp_2390 (GT87); cgp_2389 (GT87); cgp_2385 (GT87); and cgp_3164.

In embodiments, *corynebacterium* is defective for functional expression of one glycosyltransferase involved in *corynebacterial* cell wall biosynthesis selected from the group comprising or consisting of cgp_3313 (MrcB; GT51), cgp_0336 (PonA; GT51); cgp_3166 (GT4); cgp_2400 (GT4); cgp_1876 (GT4); cgp_1268 (GlgA; GT4); cgp_0554 (GT4); cgp_3191 (GlfT; GT2); cgp_1672 (PpmC; GT2); cgp_1180 (GT2); cgp_0848 (WbbL; GT2); cgp_0730 (GT2); cgp_0396 (GT2); cgp_0394 (GT2); cgp_0246 (GT2); cgp_0163 (GT2); cgp_2393 (GT87,GT87); cgp_2390 (GT87); cgp_2389 (GT87); cgp_2385 (GT87); and cgp_3164.

In embodiments, the one or more glycosyltransferases are selected from the group comprising or consisting of cgp_0336 (GT51), cgp_2385(GT87), cgp_1672 (PpmC; GT2); cgp_2390(GT87), cgp_2393(GT87), cgp_0554(GT4), cgp_1876 (GT4), cgp_1877 (GT4), cgp_2400 (GT4) and cgp_3191 (GT2).

In preferred embodiments, the *corynebacteria* of the invention are defective for cgp_3164. In preferred embodiments, the *corynebacteria* of the invention are defective for cgp_0554. In preferred embodiments, the *corynebacteria* of the invention are defective for cgp_2385. In preferred embodiments, the *corynebacteria* of the invention are defective for cgp_3191. In preferred embodiments, the *corynebacteria* of the invention are defective for cgp_0336.

In preferred embodiments, the one or more glycosyltransferases are selected from the group comprising or consisting of cgp_0336 (GT51), cgp_2385(GT87), cgp_0554(GT4), cgp_3164 and cgp_3191 (GT2).

In a further aspect, the invention relates to a method for producing fucosyllactose, the method comprising culturing a genetically modified *corynebacterium* of the present invention comprising the transgenes for fucosyllactose production as described herein in a medium supplemented with lactose.

In embodiments of the method of the invention, the culture medium additionally comprises glucose.

Preferably, the culture medium of the present invention does not comprise antibiotics.

Any features of the invention described in the context of the bacteria of the invention are herewith also disclosed in the context of the method of the invention and the other way around.

### Aspects of the invention relating to genetically modified corynebacterium with one or more defective glycosyltransferases involved in corynebacterial cell wall biosynthesis

In a further aspect, the invention relates to a genetically modified (or engineered) *corynebacterium* for production of biomolecules, characterized in that the *corynebacterium* is defective for functional expression of one or more glycosyltransferases involved in *corynebacterial* cell wall biosynthesis.

Surprisingly, it was found out that deficiency in glycosyltransferases involved in *corynebacterial* cell wall biosynthesis does not significantly impair the growth and efficiency of intracellular metabolic pathways, in particular not of an introduced fucosyllactose biosynthesis pathway.

Corynebacterial cell wall is multilayered, the phospholipidic cytoplasmic membrane is surrounded by a thick arabinogalactan-peptidoglycan sheath covalently attached to an outer membrane called mycomembrane. The mycomembrane is a complex ensemble of mycolic acids, phospholipids, lipoglycans and proteins forming a hydrophobic bilayer. It is a major permeability barrier for the transport of small hydrophilic solutes and antimicrobial compounds in genus Corynebacteria.

When testing FL production in Corynebacterium, it was found that efflux of 2FL in Corynebacterium is hampered significantly by the bacterial cell wall. Whole cell strain producing FL intracellularly in higher quantities results in accumulation of metabolic intermediates known to inhibit rate limiting genes such as ManB and GMD of the pathway. This might lead to metabolic burden on the cell and thus growth arrest or inefficient synthesis of FL. Such Inefficient fermentation and recovery processes consequently increase economic costs to scale up. Thus, it was investigated whether membrane fluidity could be improved by systematically deleting glycosyltransferases involved in the formation of the several layers of the Corynebacterial cell wall, and whether such deletions have an effect on efflux of biomolecules such as Fucosyllactose from these genetically modified Corynebacteria.

Importantly, however, it was surprisingly found that such a functional deficiency that can be caused by inactivation of one or more glycosyltransferases involved in *corynebacterial* cell wall biosynthesis reduces the foam production of *corynebacterial* cultures during production processes and therefore make such processes much easier to handle. Therefore, it is also possible to scale up the volumes of the bacterial cultures, since no additional space in the culture vessels/containers has be reserved for foam that is produced during the culture process. Also, foaming can lead to contamination and loss of material, either of which will reduce yields.

Foaming is a serious problem in corynebacterial fermentations, and a critical factor to be monitored and controlled by adding antifoam agents manually in lab scale bioprocess experiments. Process optimization accompany controlling both aeration and agitation to minimize foaming. Agitation and aeration in a stirred tank bioreactor always cause foaming, uncontrolled foaming resulting in the loss of aseptic conditions; the fermenter becomes contaminated and microorganisms are released into the environment. Additionally, foam formation may cause serious operational difficulties by blocking air filters in aerated stirred tank bioreactors, especially in high cell density cultivations. Foam formation is generally attributed to the increase in viscosity of the media due to increase in denatured proteins/peptides and surface-active agents formed during culture process and cell lysis at the air liquid interface. However additional factors might contribute in corynebacterial bioprocess with its outer membrane significantly composed of hydrophobic and amphiphilic constituents such as mycolic acids and phospholipids.

Furthermore, reduced foaming facilitates efficient oxygen exchange during fermentation. Growth of aerobic organisms require a sufficient concentration of dissolved oxygen in the medium, measured as volume of dissolved oxygen coefficient (KLa). The kLa is a measure of how much oxygen is transferred into the medium over a certain amount of time and it is not improved with increase in aeration in corynebacterial cultures, because kLa of a system can be influenced by several factors such as properties of the medium like viscosity, the presence of organisms and their by-products, excess addition of antifoam agents (Çalιk G, Ünlütabak F, Ozdamar TH. Product and by-product distributions in glutamic acid fermentation by Brevibacterium flavum: effects of the oxygen transfer. J Biochem Eng J. 2001;9:91-101; Stanbury, P. F., Whitaker, A., & Hall, S. J. (2013). Principles of fermentation technology. Elsevier). Foaming significantly reduce kLa, and oxygen transfer rate (OTR) depends upon the kLa, (Routledge, S. J. (2012). Beyond defoaming: the effects of antifoams on bioprocess productivity. Comput. Struct. Biotechnol. J. 3. doi:10.5936/csbj.201210014). Foaming also produce more waste and reduce the amount of medium and carbon sources available for bacterial growth.

There are basically three methods used to control foam production: media modification, mechanical foam-breaking devices, or the automatic addition of chemical antifoam agents. If foaming is minimized, then throughputs can be increased. Various procedures have been used in industry to reduce foam formation rate, with each of them having its own advantages and disadvantages.

Strain improvement is a vital part of process development in most fermentation industries. It provides a means by which production costs can be reduced through increases in productivity by optimal utilization of carbon sources and reduction of manufacturing costs. As described herein, surprisingly such desirable characteristics, in particular reduced foaming of Corynebacterial cultures, were identified while constructing the genetically modified strains with the aim of improving 2FL efflux.

This is advantageous not only in the context of fucosyllactose production as described herein. In contrast, these advantages of this aspect of the invention can also be harnessed for the production of other biomolecules by *corynebacteria* and in particular by *corynebacterium glutamicum,* since the advantages of defects in cell wall biosynthesis are universal to such bacterial cultures and production of biomolecules by such cultures, such as for example L-glutamate and L-lysine, lactate, cadaverine, isobutanol, succinate, itaconate, plant-derived polyphenols.

Unexpectedly, the reduction of foam does not only lead to such practical advantages for the culture and production process, but it turned out that deficiency for one or more glycosyltransferases involved in *corynebacterial* cell wall biosynthesis also allows a more efficient production and purification of biomolecules by the bacteria, as exemplified for fucosyllactose in the examples below. Therefore, besides and independent from the possibility of culturing larger volumes, it is also possible to purify higher amounts of fucosyllactose from identical culture volumes in comparison to producing *corynebacteria* that are not defective for one or more glycosyltransferases involved in *corynebacterial* cell wall biosynthesis.

Therein, the one or more glycosyltransferases are preferably selected from the group comprising cgp_3313 (MrcB; GT51), cgp_0336 (PonA; GT51); cgp_3166 (GT4); cgp_2400 (GT4); cgp_1876 (GT4); cgp_1268 (GlgA; GT4); cgp_0554 (GT4); cgp_3191 (GlfT; GT2); cgp_1672 (PpmC; GT2); cgp_1180 (GT2); cgp_0848 (WbbL; GT2); cgp_0730 (GT2); cgp_0396 (GT2); cgp_0394 (GT2); cgp_0246 (GT2); cgp_0163 (GT2); cgp_2393 (GT87,GT87); cgp_2390 (GT87); cgp_2389 (GT87); cgp_2385 (GT87); and cgp_3164.

In embodiments, *corynebacterium* is defective for functional expression of one glycosyltransferase involved in *corynebacterial* cell wall biosynthesis selected from the group comprising or consisting of cgp_3313 (MrcB; GT51), cgp_0336 (PonA; GT51); cgp_3166 (GT4); cgp_2400 (GT4); cgp_1876 (GT4); cgp_1268 (GlgA; GT4); cgp_0554 (GT4); cgp_3191 (GlfT; GT2); cgp_1672 (PpmC; GT2); cgp_1180 (GT2); cgp_0848 (WbbL; GT2); cgp_0730 (GT2); cgp_0396 (GT2); cgp_0394 (GT2); cgp_0246 (GT2); cgp_0163 (GT2); cgp_2393 (GT87,GT87); cgp_2390 (GT87); cgp_2389 (GT87); cgp_2385 (GT87); and cgp_3164.

In embodiments, the one or more glycosyltransferases are selected from the group comprising or consisting of cgp_0336 (GT51), cgp_2385(GT87), cgp_1672 (PpmC; GT2); cgp_2390(GT87), cgp_2393(GT87), cgp_0554(GT4), cgp_1876 (GT4), cgp_1877 (GT4), cgp_2400 (GT4) and cgp_3191 (GT2).

In preferred embodiments, the *corynebacteria* of the invention are defective for cgp_3164. In preferred embodiments, the *corynebacteria* of the invention are defective for cgp_0554. In preferred embodiments, the *corynebacteria* of the invention are defective for cgp_2385.ln preferred embodiments, the *corynebacteria* of the invention are defective for cgp_3191. In preferred embodiments, the *corynebacteria* of the invention are defective for cgp_0336.

In preferred embodiments, the one or more glycosyltransferases are selected from the group comprising or consisting of cgp_0336 (GT51), cgp_2385(GT87), cgp_0554(GT4), cgp_3164 and cgp_3191 (GT2).

In embodiments of the invention, the one or more glycosyltransferases are selected from the group comprising or consisting of cgp_3313 (MrcB; GT51), cgp_0336 (PonA; GT51); cgp_3166 (GT4); cgp_2400 (GT4); cgp_1876 (GT4); cgp_1268 (GlgA; GT4); cgp_0554 (GT4); cgp_3191 (GlfT; GT2); cgp_1672 (PpmC; GT2); cgp_1180 (GT2); cgp_0848 (WbbL; GT2); cgp_0730 (GT2); cgp_0396 (GT2); cgp_0394 (GT2); cgp_0246 (GT2); cgp_0163 (GT2); cgp_2393 (GT87,GT87); cgp_2390 (GT87); cgp_2389 (GT87); cgp_2385 (GT87); and cgp_3164.

In embodiments, the one or more glycosyltransferases are selected from the group comprising or consisting of cg0336 (GT51), cg2385(GT87), cgp_1672 (PpmC; GT2); cg2390(GT87), cg2393(GT87), cg0554(GT4), cg1876 (GT4), cg1877 (GT4), Cg2400 (GT4) and cgp_3191 (GT2).

In preferred embodiments, the *corynebacterium* is *corynebacterium glutamicum.*

In embodiments, the *corynebacterium* is suitable to produce fucosyllactose.

In embodiments, the genetically modified *corynebacterium* of the invention exports fucosyllactose.

In embodiments of the invention, the genetically modified *corynebacterium* is engineered to export fucosyllactose into the medium when grown at appropriate culture conditions. It is understood that export of FL relates to any kind of process, active or passive, that leads to FL being present outside the bacteria in the culture medium.

In embodiments, the *corynebacterium* has been modified to express a permease for lactose import, such as lactose permease (LacY), GDP-D-mannose-4,6-dehydratase (GMD), GDP-L-fucose synthase (WcaG) and fucosyltransferase (FucT) from exogenous nucleic acid sequences.

In embodiments, the genetically modified *corynebacterium* additionally comprise exogenous nucleic acids for expression of phosphomannomutase (ManB) and GTP-mannose-1-phosphate guanylyltransferase (ManC).

In embodiments, one or more of the exogenous nucleic acid sequences of the genetically modified bacteria are present in the bacterium as a plasmid. In embodiments, all of the exogenous nucleic acid sequences of the genetically modified bacteria are present in the bacterium as a plasmid.

In embodiments, one or more of the exogenous nucleic acid sequences of the genetically modified bacteria are chromosomally integrated. In embodiments, all the exogenous nucleic acid sequences of the genetically modified bacteria are chromosomally integrated.

In embodiments, the exogenous nucleic acid sequences encoding LacY, GMD, WcaG and FucT are chromosomally integrated. In embodiments, ManB ManC are chromosomally integrated.

All individual features disclosed and described above in the context of the genetically modified *corynebacterium* of the invention with chromosomally integrated transgenes required for production of fucosyllactose are herewith also disclosed in the context of the genetically modified *corynebacterium* of the invention for production of biomolecules, which is characterized in that the *corynebacterium* is defective for functional expression of one or more glycosyltransferases involved in *corynebacterial* cell wall biosynthesis. The same holds true for a method of producing biomolecules using the bacteria of the present invention.

### DETAILED DESCRIPTION OF THE INVENTION

All cited documents of the patent and non-patent literature are hereby incorporated by reference in their entirety.

In one aspect, the present invention relates to genetically modified (or engineered) *corynebacterium* for production of fucosyllactose, wherein the *corynebacterium* has been modified to express a permease for lactose import, GDP-D-mannose-4,6-dehydratase (GMD), GDP-L-fucose synthase (WcaG) and fucosyltransferase (FucT) from exogenous nucleic acid sequences, characterized in that the exogenous nucleic acid sequences encoding a permease for lactose import, GMD, WcaG and FucT are chromosomally integrated.

*Corynebacterium* is a genus of bacteria that are Gram-positive and aerobic. They are bacilli (rod-shaped), and in some phases of life they are club-shaped, which inspired the genus name (*coryneform* means "club-shaped"). They are widely distributed in nature in the microbiota of animals (including the human microbiota) and are mostly innocuous, most commonly existing in commensal relationships with their hosts. *Corynebacteria* are useful in industrial settings in particular *Corynebacterium glutamicum.*

16 conserved signature proteins, which are uniquely found in *Corynebacterium* species, have been identified. Three of the conserved signature proteins have homologs found in the genus Dietzia, which is believed to be the closest related genus to *Corynebacterium.* In phylogenetic trees based on concatenated protein sequences or 16S rRNA, the genus *Corynebacterium* forms a distinct clade, within which is a distinct subclade, cluster I. The cluster is made up of the species *C*. *diptheriae, C. pseudotuberculosis, C. ulcerans, C. aurimucosum, C. glutamicum,* and *C*. *efficiens.* This cluster is distinguished by several conserved signature indels, such as a two-amino-acid insertion in LepA and a seven- or eight-amino-acid insertions in RpoC. Also, 21 conserved signature proteins are found only in members of cluster I. Another cluster has been proposed, consisting of *C. jeikeium* and *C*. *urealyticum,* which is supported by the presence of 19 distinct conserved signature proteins which are unique to these two species. *Corynebateria* have a high G+C content ranging from 46-74 mol%.

*Corynebateria* are gram-positive, catalase-positive, non-spore-forming, non-motile, rod-shaped bacteria that are straight or slightly curved. Metachromatic granules are usually present representing stored phosphate regions. Their size falls between 2 and 6 µm in length and 0.5 µm in diameter. The bacteria group together in a characteristic way, which has been described as the form of a "V", "palisades", or "Chinese characters". They may also appear elliptical. They are aerobic or facultatively anaerobic, chemoorganotrophs. They are pleomorphic through their lifecycles, they occur in various lengths, and they frequently have thickenings at either end, depending on the surrounding conditions.

The *Corynebateria* cell wall is distinctive, with a predominance of mesodiaminopimelic acid in the murein wall and many repetitions of arabinogalactan, as well as corynemycolic acid (a mycolic acid with 22 to 26 carbon atoms), bound by disaccharide bonds called L-Rhap-(1 → 4)--D-GlcNAc-phosphate. These form a complex commonly seen in *Corynebacterium* species: the mycolyl-AG-peptidoglican (mAGP).

*Corynebacteria* grow slowly, even on enriched media. In terms of nutritional requirements, all need biotin to grow. Some strains also need thiamine and PABA. Some of the *Corynebacterium* species with sequenced genomes have between 2.5 and 3.0 million base pairs. The bacteria grow in Loeffler's medium, blood agar, and trypticase soy agar (TSA). They form small, grayish colonies with a granular appearance, mostly translucent, but with opaque centers, convex, with continuous borders. The color tends to be yellowish-white in Loeffler's medium. In TSA, they can form grey colonies with black centers and dentated borders that look similar to flowers (*C. gravis*), or continuous borders (*C. mitis*), or a mix between the two forms (*C. intermedium*).

Nonpathogenic species of *Corynebacterium* are used for very important industrial applications, such as the production of amino acids, nucleotides, and other nutritional factors; bioconversion of steroids; degradation of hydrocarbons; cheese aging; and production of enzymes. Some species produce metabolites like antibiotics: bacteriocins of the corynecin-linocin type, antitumor agents, among others. One of the most studied species is *C. glutamicum,* whose name refers to its capacity to produce glutamic acid in aerobic conditions. Species of *Corynebacterium* and in particular *C. glutamicum* have been used in the mass production of various amino acids including glutamic acid, a food additive that is made at a rate of 1.5 million tons/ year. The metabolic pathways of *Corynebacterium* have been further manipulated to produce lysine and threonine. L-Lysine production is specific to *C. glutamicum* in which core metabolic enzymes are manipulated through genetic engineering to drive metabolic flux towards the production of NADPH from the pentose phosphate pathway, and L-4-aspartyl phosphate, the commitment step to the synthesis of L-lysine, lysC, dapA, dapC, and dapF. These enzymes are up regulated in industry through genetic engineering to ensure adequate amounts of lysine precursors are produced to increase metabolic flux. Unwanted side reactions such as threonine and asparagine production can occur if a buildup of intermediates occurs, so scientists have developed mutant strains of *C. glutamicum* through PCR engineering and chemical knockouts to ensure production of side-reaction enzymes are limited. Many genetic manipulations conducted in industry are by traditional cross-over methods or inhibition of transcriptional activators.

Unlike gram-negative bacteria, the gram-positive *Corynebacterium* species lack lipopolysaccharides that function as antigenic endotoxins in humans, which is highly advantageous to produce biomolecules intended for consumption by humans.

*Corynebacterium* species include nonlipophilic *Corynebacteria* that may be classified as fermentative and nonfermentative. Fermentative *corynebacteria* include *Corynebacterium diphtheriae* group, *Corynebacterium xerosis* and *Corynebacterium striatum, Corynebacterium minutissimum, Corynebacterium amycolatum, Corynebacterium glucuronolyticum, Corynebacterium argentoratense, Corynebacterium matruchotii, Corynebacterium glutamicum, Corynebacterium sp.* Nonfermentative *corynebacteria* include *Corynebacterium afermentans subsp. Afermentans, Corynebacterium auris, Corynebacterium pseudodiphtheriticum, Corynebacterium propinquum.* Lipophilic *Corynebacteria* include Corynebacterium uropygiale, Corynebacterium jeikeium, *Corynebacterium urealyticum, Corynebacterium afermentans subsp. Lipophilum, Corynebacterium accolens, Corynebacterium macginleyi, CDC coryneform* groups F-1 and G, *Corynebacterium bovis.* This list of *Corynebacteria* is non limiting for the scope of the invention.

In embodiments, the *Corynebacterium* of the invention is a non-pathogenic species or strain. In embodiments, the *Corynebacterium* is a strain that is considered a GRAS host. In preferred embodiments, the *corynebacterium* is *C. glutamicum.*

*Corynebacterium glutamicum* (previously known as *Micrococcus glutamicus*) is a Gram-positive, rod-shaped bacterium that is used industrially for large-scale production of amino acids. While originally identified in a screen for organisms secreting L-glutamate, mutants of *C. glutamicum* have also been identified that produce various other amino acids. Due to its industrial importance, several clones of *C. glutamicum* have been sequenced. Furthermore, small RNA data was obtained by RNA-Seq in C. glutamicum ATCC 13032. *Corynebacterium glutamicum* ATCC 14067 was previously known as *Brevibacterium flavum.*

Furthermore, with the sequencing of the whole genome of *C. glutamicum,* new methods such as proteomics, metabolomics and transcriptome analyses were established that have led to a variety of approaches in metabolic engineering to rationally construct/improve production strains.

Stable maintenance of expression plasmids is unfavorable in industrial production strains because antibiotics must be supplied to the cultivation media. Consequently, heterologous genes are integrated, or native genes are deleted in the genome of the respective organism. The insertion, deletion or substitution of genes in *C. glutamicum* is usually performed by homologous recombination using non-replicative integration vectors and genome modification can be achieved via two rounds of positive selection, as is known to the skilled person. One example method of this kind of genetic modification uses the non-replicative plasmid pK19mobsacB, which can preferably be used to generate the corynebacterial of the present invention. Furthermore, targeted genome modification in *C. glutamicum* can also be performed via conjugation by using *E. coli* vectors carrying manipulated *C. glutamicum* DNA fragments.

The present invention relates to genetically modified (or genetically engineered) *Corynebacteria.* Therein, the term "genetically modified" describes bacteria whose genetic material has been modified in comparison to a naturally occurring wild type strain, for example by deleting or removing genetic elements of the wild type strain and/or by inserting additional genetic material, in particular DNA sequences, for example in form of non-integrating DNA plasmids or as DNA sequences that chromosomally integrate into the bacterial genome. Genetic modifications also include deletions of chromosomal sequences or other genetic elements of the bacteria. Possible techniques of genetically modifying *Corynebacterium* and in particular *C. glutamicum* are described herein. As used herein, transgenic bacteria are genetically modified bacteria. A transgenic bacterium is a bacterium that comprises an exogenous nucleic acid molecule for expression of a protein, which is preferably from another organism or species, for example.

In one aspect, the *corynebacterium* of the invention is to produce fucosyllactose. In preferred embodiments, the fucosyllactose comprises 2-Fucosyllactose (2-FL or 2FL). In further embodiments, fucosyllactose comprises 3-Fucosyllactose (3-FL or 3FL).

2-FL (IUPAC name (2*R*,3*R*,4*R*,5*R*)-4-[(2*S*,3*R*,4*S*,5*R*,6*R*)-4,5-dihydroxy-6-(hydroxymethyl)-3-[(2S,3S,4R,5S,6S)-3,4,5-trihydroxy-6-methyloxan-2-yl]oxyoxan-2-yl]oxy-2,3,5,6-tetrahydroxyhexanal) is an oligosaccharide, more precisely, fucosylated, neutral trisaccharide composed of L-fucose, D-galactose, and D-glucose units. It is the most prevalent human milk oligosaccharide (HMO) naturally present in human breast milk, making up about 30% of all of HMOs.

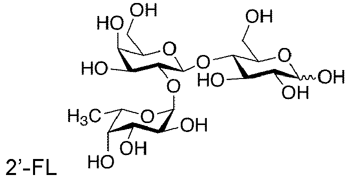

As with other oligosaccharides, a widely regarded characteristic of 2'-fucosyllactose is its ability to protect against infectious diseases namely in preventing epithelial level adhesions of toxins and pathogens. The 2FL stimulates the growth of certain Bifidobacteria and receptor analogons which lends to toxic and pathogenic protection, all this being most prevalent in infants. Among the pathogens that 2FL is known to protect against are *Campylobacter jejuni, Salmonella enterica serotype Typhimurium, Helicobacter pylori,* among others.

3-FL (3-Fucosyllactose) is one of the most abundant fucosylated HMOs, and particularly dominant in the milk of non-secretor mothers. 3-FL occurs in human milk in a concentration of 0.72 (± 0.7) g/L. While the concentration of 2-FL decrease over the lactation time, the amount of 3-FL increases in later lactation stages. The specific functional benefits of 3-FL include reducing the risk of infection by inhibiting the adhesion of pathogenic bacteria, e.g. *Pseudomonas aeruginosa* or enteropathogenic *E*. *coli* or viruses e.g. Norovirus. It also shows general ability to reduce colonization of non-beneficial bacteria, e.g. *Enterococcus faecium,* and in turn positively supporting gut health by selectively stimulating beneficial bifidobacterial. Further studies on 3-FL suggest positive effects in regulating intestinal motility.

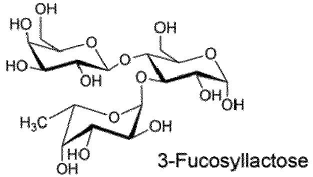

For engineering a fucosyllactose biosynthesis pathway in the *Corynebacterium,* the *Corynebacterium* of the invention can comprise an exogenous nucleic acid sequence encoding and enabling expression of a fucosyltransferase (FucT).

Fucosyltransferases belong to Leloir glycosyltransferases, as these enzymes use a nucleotide-activated sugar (GDP-fucose (guanosine diphosphate-fucose) as glycosyl donor for their reaction. In the carbohydrate active enzymes database (CAZy, http://www.cazy.org), which classifies glycosyltransferses based on amino acid sequence homology, fucosyltransferases are classified under glycosyltransferase families GT-10, GT-11, GT-23, GT-37, GT-65, GT-68 and GT-74. The FucTs relevant for HMO synthesis belong to GT-10 (α1,3-, and/or α1,4-FucTs) or GT-11 (α1,2-FucTs) and GT 74 (α1,2-FucT). Fucosyltransferases (FucTs, EC 2.4.1.x) are categorized into α1,2-, α1,3/4-, and α1,6-selective enzyme groups, depending on the site selectivity of the enzyme in the reaction with the acceptor carbohydrates. The acceptor substrate can be another sugar such as the transfer of a fucose to a core GlcNAc (N-acetylglucosamine) sugar as in the case of N-linked glycosylation, or to a protein, as in the case of O-linked glycosylation produced by O-fucosyltransferase. There are various fucosyltransferases in mammals, the vast majority of which, are located in the Golgi apparatus. The O-fucosyltransferases have recently been shown to localize to the endoplasmic reticulum (ER). (Brockhausen, Inka. "Crossroads between Bacterial and Mammalian Glycosyltransferases." Frontiers in Immunology, vol. 5, 2014, pp. 492-492; Petschacher, Barbara, and Bernd Nidetzky. "Biotechnological Production of Fucosylated Human Milk Oligosaccharides: Prokaryotic Fucosyltransferases and Their Use in Biocatalytic Cascades or Whole Cell Conversion Systems." Journal of Biotechnology, vol. 235, 2016, pp. 61-83.)

As used herein, the term "fucosyltransferase" comprises in particular "alpha-1,2-fucosyltranferase" (FucT2) and "alpha-1,3-fucosyltranferase" (FucT3). A fucosyltransferase or a nucleic acid sequence/polynucleotide encoding an fucosyltransferase refer to a glycosyltransferase that catalyzes the transfer of fucose from a donor substrate, for example, GDP-fucose, to an acceptor molecule, preferably in an alpha-1,2-linkage or alpha-1,3-linkage, respectively. The acceptor molecule can be a carbohydrate, an oligosaccharide, a protein or glycoprotein, or a lipid or glycolipid, and can be, e.g., N-acetylglucosamine, N-acetyllactosamine, galactose, fucose, sialic acid, glucose, lactose or any combination thereof. In the context of the present invention, the acceptor molecule is preferably lactose.

Preferred fucosyltransferases for use in the context of the invention comprise FucT from *Helicobacter pylori* (NCBI-Protein ID AAD29863.1, UniProt Q9X435) and FucT from *Helicobacter mustelae* (NCBI-Protein ID CBG40460.1, UniProt D3UIY5) and alpha-1,2-FucT WbsJ from *E*. *coli* O128:B12. However, Genera of organisms with know FucT activities (both α-1,2- and α-1,3 FucTs) suited for use in the context of the present invention comprise Helicobacter, Escherichia, Bacteroides, Anopheles, Apis, Caenorhabditis, Cricetulus, Danio, Rattus, Gallus, Medicago, Sus, Oryza, Physcomitrium, Gallus, Mus, Bos, Schistosoma, Canis, Vigna, Zea, Eulemur Gorilla, Hylobates, Macaca, Oryctolagus, Pan, Pongo, Thermosynechococcus, Dictyostelium , Arabidopsis, Drosophila, and Homo. A complete list of suitable FucT enzymes known so far is disclosed on www.cazy.org (Carbohydrate-Active enZYmes Database describing families of structurally-related catalytic and carbohydrate-binding modules (or functional domains) of enzymes that degrade, modify, or create glycosidic bonds), specifically http://www.cazy.org/GT10_characterized.html (for GlycosylTransferase Family 10 comprising various FucT3 from many different organisms) and http://www.cazy.org/GT11_characterized.html (for GlycosylTransferase Family 11 comprising various FucT2 from many different organisms).

Few FucTs from prokaryotic and eukaryotes have been characterized. Examples include α-FucTs from *H. pylori, H. Mustulae, E. coli* O86:K62:H2, *E. coli* O86:B7, *E. coli* O128:B12, *E. coli* O127:K63(B8), *E. coli* 0126 and *Thermosynechoccus elongatus, and* α-1,3/4-FucTs from *H*. *pylori, H. hepaticus and B. fragilis.*

In embodiments, the one or more FucT expressed by the *corynebacterium* of the invention is selected from the group comprising α-FucTs from *H. pylori, H. Mustulae, E. coli* O86:K62:H2, *E*. *coli* O86:B7, *E. coli* O128:B12, *E. coli* O127:K63(B8), *E. coli* O126 and *Thermosynechoccus elongatus.* α-1,3/4-FucTs from *H. pylori, H. hepaticus and B. fragilis.*

In preferred embodiments, one or more FucT expressed by the *corynebacterium* of the invention is selected from the group comprising α-FucTs from *H. pylori, H. Mustulae, E. coli* O126 and *Thermosynechoccus elongatus* and α-1,3/4-FucTs from *H. pylori, H. hepaticus and B. fragilis.*

In preferred embodiments, one or more FucT expressed by the *corynebacterium* of the invention is selected from the group comprising α-FucTs from *H. pylori, H. Mustulae, E. coli* O126 and *Thermosynechoccus elongatus.*

In preferred embodiments, one or more FucT expressed by the *corynebacterium* of the invention is selected from the group comprising α-1,3/4-FucTs from *H. pylori, H. hepaticus and B. fragilis.*

Furthermore, for engineering a fucosyllactose biosynthesis pathway in the *Corynebacterium,* the *Corynebacterium* comprise in embodiments an exogenous nucleic acid sequences encoding and enabling expression of GDP-D-mannose-4,6-dehydratase (GMD).

A GDP-mannose 4,6-dehydratase (GMD) is an enzyme that catalyzes the chemical reaction GDP-mannose ⇄ GDP-4-dehydro-6-deoxy-D-mannose + H₂O. Hence, this enzyme has one substrate, GDP-mannose, and two products, GDP-4-dehydro-6-deoxy-D-mannose and H₂O. Known GMDs from different organisms that can be used in the context of the present invention are disclosed on https://enzyme.expasy.org/EC/4.2.1.47), for example.

GMD belongs to the family of lyases, specifically the hydro-lyases, which cleave carbon-oxygen bonds. The systematic name of this enzyme class is GDP-mannose 4,6-hydro-lyase (GDP-4-dehydro-6-deoxy-D-mannose-forming). Other names in common use include guanosine 5'-diphosphate-D-mannose oxidoreductase, guanosine diphosphomannose oxidoreductase, guanosine diphosphomannose 4,6-dehydratase, GDP-D-mannose dehydratase, GDP-D-mannose 4,6-dehydratase, Gmd, and GDP-mannose 4,6-hydro-lyase. This enzyme participates in fructose and mannose metabolism. It employs one cofactor, NAD+.

GMD is present in the GDP-mannose-dependent de novo pathway which provides GDP-Fucose. In the pathway the enzyme is in an intermediate step that converts GDP-Mannose to GDP-4-dehydro-6-deoxy-D-mannose which is then subsequently converted into GDP-Fucose. The product of this pathway is used by fucosyltransferases.

For engineering a fucosyllactose biosynthesis pathway in the *Corynebacterium,* the *Corynebacterium* comprise in embodiments an exogenous nucleic acid sequences encoding and enabling expression of GDP-L-fucose synthase (WcaG).

A GDP-L-fucose synthase (WcaG) is an enzyme that catalyzes the following chemical reaction:

GDP-4-dehydro-6-deoxy-D-mannose + NADPH + H+ ⇄ GDP-L-fucose + NADP+

Thus, the three substrates of WcaG are GDP-4-dehydro-6-deoxy-D-mannose, NADPH, and H+, whereas its two products are GDP-L-fucose and NADP+. WcaG belongs to the family of oxidoreductases, specifically those acting on the CH-OH group of donors with NAD+ or NADP+ as acceptor. The systematic name of this enzyme class is GDP-L-fucose:NADP+ 4-oxidoreductase (3,5-epimerizing). This enzyme is also called GDP-4-keto-6-deoxy-D-mannose-3,5-epimerase-4-reductase. WcaG participates in the GDP-mannose-dependent de novo pathway which provides GDP-Fucose. Therein, WcaG converts GDP-4-dehydro-6-deoxy-D-mannose into GDP-Fucose and therefore acts downstream of GMD. Known GMDs and WcaGs from different organisms that can be used in the context of the present invention are disclosed on https://enzyme.expasy.org/EC/4.2.1.47 and https://enzyme.expasy.org/EC/1.1.1.271, respectively. for example.

Furthermore, in embodiments the *Corynebacterium* comprise a permease for lactose import. Such permeases comprise various lactose permeases, which are membrane proteins that are members of the major facilitator superfamily. Lactose permease can be classified as a symporter, which uses the proton gradient towards the cell to transport β-galactosides such as lactose in the same direction into the cell. The protein has twelve transmembrane alpha-helices and its molecular weight is about 45,000 Daltons. It exhibits an internal two-fold symmetry, relating the N-terminal six helices onto the C-terminal helices. It is encoded by the lacY gene in the lac operon of various bacteria, such as *E. coli.* The present invention is not limited to specific Lactose permeases, as long as lactose import into the *corynebacterium* can be ensured by expression of the lactose permease from the exogenous nucleic acid sequence. A skilled person can identify suitable lactose permeases by using well established functional tests. Preferred lactose permeases include LacY from *E*. *coli* and LacS from *Lactobacillus delbrueckii* and Streptococcus thermophilus as disclosed in the examples.

In embodiments, the *Corynebacterium* comprise exogenous nucleic acid sequences encoding and enabling expression of phosphomannomutase (ManB).

A phosphomannomutase (ManB) is an enzyme that catalyzes the following chemical reaction:

alpha-D-mannose 1-phosphate ⇄ D-mannose 6-phosphate

Hence, this enzyme has one substrate, alpha-D-mannose 1-phosphate, and one product, D-mannose 6-phosphate. ManB belongs to the family of isomerases, specifically the phosphotransferases (phosphomutases), which transfer phosphate groups within a molecule. The systematic name of this enzyme class is alpha-D-mannose 1,6-phosphomutase. Other names in common use include mannose phosphomutase, phosphomannose mutase, and D-mannose 1,6-phosphomutase. ManB participates in fructose and mannose metabolism. It has 2 cofactors: D-glucose 1,6-bisphosphate, and D-Mannose 1,6-bisphosphate. Known ManB variants from different organisms that can be used in the context of the present invention are disclosed on https://enzyme.expasy.org/EC/5.4.2.8, for example.

In embodiments, the *Corynebacterium* comprise exogenous nucleic acid sequences encoding and enabling expression of GTP-mannose-1-phosphate guanylyltransferase (ManC).

GTP-mannose-1-phosphate guanylyltransferase (ManC) (or mannose-1-phosphate guanylyltransferase) is an enzyme that catalyzes the following chemical reaction:

GTP + alpha-D-mannose 1-phosphate ⇄ diphosphate + GDP-mannose

Thus, the two substrates of this enzyme are GTP and alpha-D-mannose 1-phosphate, whereas its two products are diphosphate and GDP-mannose. This enzyme belongs to the family of transferases, specifically those transferring phosphorus-containing nucleotide groups (nucleotidyltransferases). The systematic name of this enzyme class is GTP: alpha-D-mannose-1-phosphate guanylyltransferase. Other names in common use include GTP-mannose-1-phosphate guanylyltransferase, PIM-GMP (phosphomannose isomerase-guanosine 5'-diphospho-D-mannose, pyrophosphorylase), GDP-mannose pyrophosphorylase, guanosine 5'-diphospho-D-mannose pyrophosphorylase, guanosine diphosphomannose pyrophosphorylase, guanosine triphosphate-mannose 1-phosphate guanylyltransferase, and mannose 1-phosphate guanylyltransferase (guanosine triphosphate). This enzyme participates in fructose and mannose metabolism. Known ManC variants from different organisms that can be used in the context of the present invention are disclosed on https://enzyme.expasy.org/EC/2.7.7.13, for example. Preferred nucleic acid sequences of the invention encoding preferred variants of the enzymes that can be exogenously expressed in embodiments of the bacteria of the invention are provided in Table 1.

**Table 1. Preferred nucleic acid sequences encoding transgenes of the corynebacterium of the invention.**

| | |
|---|---|
| SEQ ID NO 1: ManB (corresponding to NCBI-ProteinID ADT75633, and UniProt E0J145) | ATGTCGAGTTTAACCTGCTTTAAAGCCTATGATATTCGCGGGAAA TTAGGCGAAGAACTGAATGAAGATATCGCCTGGCGTATTGGGCG TGCCTATGGCGAATTTCTCAAACCGAAAACCATTGTGTTAGGCGG TGACGTCCGCCTCACCAGCGAAACCTTAAAACTGGCGCTGGCGA GAGGTTTACAGGATGCGGGCGTCGATGTGCTGGATATCGGCATG TCCGGCACCGAAGAGATCTATTTCGCCACGTTCCATCTCGGCGT GGATGGCGGCATCGAAGTTACCGCCAGCCATAATCCGATGGATT ATAACGGCATGAAGCTGGTTCGCGAGGGGGCTCGCCCGATCAG CGGAGATACCGGACTGCGCGACGTCCAGCGTCTGGCTGAAGCC AACGACTTTCCTCCCGTCGATGAAACCAAACGCGGTCGCTATCAG CAAATCAACCTGCGTGACGCTTACGTTGATCACCTGTTCGGTTAT ATCAATGTCAAAAACCTCACGCCGCTCAAGCTGGTGATCAACTCC GGGAACGGCGCAGCGGGTCCGGTGGTGGACGCTATCGAAGCCC GCTTTAAAGCCCTCGGCGCACCTGTGGAATTGATCAAAGTGCACA ACACGCCGGACGGCAATTTCCCCAACGGTATTCCTAACCCGCTG CTGCCGGAATGTCGCGACGACACCCGCAATGCGGTCATCAAACA CGGCGCGGATATGGGCATTGCCTTTGATGGCGATTTTGACCGCT GTTTCCTGTTTGACGAAAAAGGGCAGTTTATCGAGGGCTACTACA TTGTCGGCCTGCTGGCAGAAGCGTTCCTCGAAAAAAATCCCGGC GCGAAGATCATCCACGATCCACGTCTCTCCTGGAATACCGTTGAT GTGGTGACCGCCGCGGGCGGCACACCGGTGATGTCGAAAACCG GACACGCCTTTATTAAAGAACGTATGCGCAAGGAAGACGCCATCT ACGGTGGCGAAATGAGCGCCCACCACTATTTCCGTGATTTCGCTT ACTGCGACAGCGGCATGATCCCGTGGCTGCTGGTCGCCGAACTG GTGTGTCTGAAAGGAAAAACGCTGGGCGAACTGGTGCGCGACCG GATGGCAGCGTTTCCGGCAAGCGGTGAGATCAACAGCAAACTGG CGCAACCCGTTGAGGCGATTAATCGCGTCGAACAGCATTTTAGC CGCGAGGCGCTGGCGGTGGATCGCACCGATGGCATCAGCATGA CCTTTGCCGACTGGCGCTTTAACCTGCGCTCCTCCAACACCGAA CCGGTGGTGCGGTTGAATGTGGAATCGCGCGGTGATGTGCCGCT GATGGAAGAAAGACAAAACTTATCCTTGCGTTATTGAACAAGTA A |
| SEQ ID NO 2: ManC (corresponding to NCBI-ProteinID ADT75634, and UniProt E8PZ98) | ATGAACAATAATAAAATTATTACACCTATCATTATGGCAGGTGGTT CAGGCAGTCGGTTGTGGCGACTATCAAGAATTCTCTATCCGAAAC AATTTCTTAGCCTAAACGGTAGTCATACCATGCTTCAAACAACGG CTAATCGTCTGGATGGTTTGGATTGTACCAACCCTTATGTCATTTG TAATGAACAACACCGCTTTATAGTTGCTGAACAGCTTAGAAAAATC GATAGATTGACTTCAAAGAATATCATCCTTGAGCCTGTTGGGCGT AACACTGCCCCTGCAATTGCATTAGCGGCGTTGCTGATGTCTAAG TCTGATAAAAGTGCAGATGATCTTATGCTCGTACTGGCTGCAGAT CACGTTATATTAGATGAAGAAAAATTTTGTAACGCTGTTAGATCGG CAATTCCATACGCTGCTGATGGGAAATTGGTAACATTTGGTATAAT TCCAGACAAAGCAGAAACTGGTTATGGTTATATACATCGAGGACA ATATATTAATCAGGAAGATTCGGATGCATTTATAGTGTCATCATTT GTTGAAAAGCCAAATCATGAGACAGCCACTAAATATCTTGCTTCC |
| | GGTGAGTATTATTGGAATAGCGGTATGTTTTTGTTTAGTGCAAATC GTTATATAGAGGAACTTAAACAATTTCGGCCTGATATTTTATCCGC TTGTGAAAAAGCAATTGCTTCAGCGAACTTTGACCTTGATTTTGTG CGTTTAGATGAAAGTTCTTTCTCTAAGTGTCCTGAAGAGTCAATTG ATTACGCTGTAATGGAAAAAACAAAAGACGCAATTGTTATTCCAAT GGATGCTGGCTGGAGTGATGTCGGTTCATGGTCTTCTCTTTGGG AAATTAATGATAAAGACTCAGACGGCAACGTAATAGTTGGGGATA TTTTCTCTCATGAAACAAAGAATTCTTTCATATATGCCGAATCGGG AATTGTTGCTACAGTGGGAGTGGAAAATTTAGTTGTTGTCCAAAC AAAGGATGCCGTTCTTGTCTCAGAGAGAAATAAAGTTCAGGATGT AAAGAAAATAGTCGAACAAATTAAAAATTCAGGTCGTAGCGAGCA TTATGTTCATCGCGAAGTATATCGTCCTTGGGGTAAATATGATTCC ATTGACACAGGGGAGCGTTATCAGGTCAAACGTATAACAGTAAAT CCTGGTGAAGGACTTTCTTTACAAATGCACCATCATAGGGCAGAA CATTGGATCATAGTTTCTGGAACTGCAAAGGTGACTATAGGTTCT GAAACTAAGATTCTTAGCGAAAATGAATCTGTTTACATACCTCTTG GTGTAATACACTGCTTGGAAAATCCAGGGAAAATTCCTCTTGATTT AATTGAAGTTCGTTCTGGATCTTATTTAGAAGAAGACGATGTTATC CGTTTTCAGGACCGATATGGTCGCAGCTAA |
| SEQ ID NO 3: GMD from E. coli (GMD_EC) (corresponding to NCBI-ProteinID ADT75653, and UniProt E0J125) | ATGTCAAAAGTCGCTCTCATCACCGGTGTAACCGGACAAGACGG TTCTTACCTGGCAGAGTTTCTGCTGGAAAAAGGTTACGAGGTGCA TGGTATTAAGCGTCGTGCATCGTCCTTCAACACCGAGCGCGTGG ATCACATTTATCAGGATCCGCACACCTGCAACCCGAAATTCCATC TGCATTATGGCGACCTGAGTGATACCTCCAACCTGACGCGCATTT TGCGTGAAGTGCAGCCGGATGAAGTGTACAACCTGGGCGCAATG AGTCACGTTGCGGTTTCTTTTGAGTCACCGGAATATACCGCAGAC GTCGATGCGATGGGTACGCTGCGCCTGCTGGAGGCGATCCGCTT CCTCGGTCTGGAAAAGAAAACCCGTTTCTATCAGGCTTCCACCTC TGAACTGTACGGTCTGGTGCAGGAAATTCCGCAGAAAGAAACCA CGCCGTTCTACCCGCGATCTCCGTATGCGGTCGCCAAACTGTAT GCCTACTGGATCACCGTTAACTACCGCGAATCCTACGGCATGTAC GCCTGTAACGGTATTCTCTTCAACCATGAATCCCCGCGCCGCGG CGAAACCTTCGTTACCCGCAAAATCACCCGCGCAATCGCCAACAT CGCCCAGGGGCTGGAGTCGTGCCTGTACCTCGGCAATATGGATT CCCTGCGTGACTGGGGCCACGCCAAAGACTACGTAAAAATGCAG TGGATGATGCTGCAACAGGAACAGCCGGAAGATTTCGTTATCGC GACCGGCGTTCAGTACTCCGTGCGTCAGTTCGTGGAAATGGCAG CGGCACAGCTGGGCATCAAACTGCGCTTTGAAGGCACGGGTGTT GAAGAGAAGGGCATTGTGGTTTCCGTCACCGGGCATGACGCGCC GGGCGTTAAACCGGGTGATGTGATTATCGCCGTTGATCCGCGTT ACTTCCGTCCGGCTGAAGTTGAAACGCTGCTCGGCGACCCGACC AAAGCGCACGAAAAACTGGGCTGGAAACCGGAAATCACCCTCAG AGAGATGGTGTCTGAAATGGTGGCTAATGACCTCGAAGCGGCGA AAAAACACTCTCTGCTGAAATCTCACGGCTACGACGTGGCGATCG CGCTGGAGTCATAA |
| SEQ ID NO 4: WcaG from E. coli (WcaG_EC) (corresponding to NCBI-ProteinID ADT75652, and UniProt E0J126) | ATGAGTAAACAACGCATTTTTATCGCTGGCCATCGTGGATGGTC GGTTCCGCCATCACGCGGCAGCTCGAACAGCGCGGTGATGTGG AACTGGTATTACGCACCCGCGACGAGCTGAACCTGCTGGACAGC CGCGCGGTGCATGATTTCTTTGCCAGCGAACGCATTGACCAGGT CTATCTGGCGGCGGCGAAAGTGGGCGGCATTGTTGCCAACAACA CCTATCCGGCGGATTTCATCTACCAGAACATGATGATTGAGAGCA ACATCATTCACGCCGCGCATCAGAACGACGTGAACAAACTGCTGT |
| | TTCTTGGATCGTCCTGTATCTACCCGAAACTGGCAAAACAGCCGA TGGCAGAAAGCGAGTTATTGCAGGGCACGCTGGAGCCGACCAAC GAGCCTTACGCCATTGCCAAAATCGCCGGGATCAAACTGTGCGA ATCATACAACCGCCAGTACGGACGCGATTACCGCTCAGTCATGC CGACCAACCTGTATGGCCCGCATGACAACTTCCACCCGAGTAATT CGCATGTGATCCCTGCATTGCTGCGTCGCTTCCACGAGGCGACG GCACAGAATGCACCGGACGTGGTGGTATGGGGCAGCGGTACAC CGATGCGTGAATTCCTGCACGTCGATGATATGGCGGCGGCGAGC ATTCATGTCATGGAGCTGGCGCACGAAGTCTGGCTGGAGAACAC CCAGCCGATGCTGTCGCACATTAACGTCGGCACGGGCGTTGACT GCACTATCCGCGAGCTGGCGCAAACCATCGCCAAAGTGGTGGGT TACAAAGGTCGGGTGGTTTTTGATGCCAGCAAACCGGATGGTAC GCCGCGCAAACTGCTTGATGTGACGCGCCTGCATCAGCTTGGCT GGTATCACGAAATCTCACTGGAAGCGGGGCTTGCCAGCACTTAC CAGTGGTTCCTTGAGAATCAAGACCGCTTTCGGGGGTAA |
| SEQ ID NO 5: Gmd from Bacteroides fragilis (Gmd_BF) (corresponding to NCBI-ProteinID CAH07586, and UniProt Q5LE66) | ATGATTGACAGAATGGATAAAAACGCAAAAATTTATGTAGCCGGA CACCACGGACTGGTGGGTTCGGCTATATGGAAAAATCTGCAGGA AAAGGGGTATACGAATCTGGTGGACGCACACATAAGGAACTGG ACTTATTGGACGGTGCGACCGTAAAGCAGTTTTTTGATGAGGAAA TGCCGGAGTACGTGTTTTTGGCTGCCGCTTTTGTCGGAGGAATCA TGGCCAATAGTATCTACCGTGCGGACTTTATCTATAAGAACTTACA GATACAGCAGAACGTGATCGGAGAAAGTTTCCGGCATCAGGTGA AAAAACTGCTCTTTCTGGGCAGTACCTGCATCTATCCGAGGGATG CCGAACAGCCGATGAAAGAGGACGTCTTGCTCACTTCTCCACTG GAATATACCAATGAACCTTATGCCATAGCCAAAATAGCCGGACTG AAAATGTGCGAAAGCTTCAACCTGCAATATGGAACGAACTACATC GCCGTGATGCCGACCAACCTGTATGGGCCGAACGATAACTTTGA CTTGGAACGCAGTCATGTGCTGCCTGCCATGATCCGTAAAGTTCA CTTGGCACACTGCCTGAAAAAAGGAGATTGGGAGGCCGTGCGTA AAGATATGAACCTGCGTCCGGTAGAAGGCATCAGTGGTGCCAAC TCCAACGAAGAGATCCTCCGGATTCTCCGGAAATACGGCATTACT GAAACGGAGGTGACACTTTGGGGAACAGGAACGCCTCTAAGGGA ATTTCTTTGGAGTGAAGAAATGGCAGATGCCAGTGTCTTCGTAAT GGAACATGTGGACTTCAAGGATACCTACAAAACCGGTGCAAAGA CATCCGCAACTGCCACATCAATATAGGTACCGGCAAAGAAATTAC AATCCGCGAACTGGCCGGACTGATTGTAAATACAGTCGGCTATCA GGGTGAACTGACTTTTGACAGCAGTAAACCGGACGGAACCATGC GAAAACTTACCGATCCGTCGAAATTGCACAACCTCGGATGGCATC ATAAGATCGATATTGAAGAGGGGGTACAGAAAATGTACGAGTGGT ATCTGGGATAA |
| SEQ ID NO 6: WcaG from Bacteroides fragilis (WcaG_BF) (corresponding to NCBI-ProteinID CAH07585, and UniProt Q5LE67) | ATGATTGACAGAATGGATAAAAACGCAAAAATTTATGTAGCCGGA CACCACGGACTGGTGGGTTCGGCTATATGGAAAAATCTGCAGGA AAAGGGGTATACGAATCTGGTGGGACGCACACATAAGGAACTGG ACTTATTGGACGGTGCGACCGTAAAGCAGTTTTTTGATGAGGAAA TGCCGGAGTACGTGTTTTTGGCTGCCGCTTTTGTCGGAGGAATCA TGGCCAATAGTATCTACCGTGCGGACTTTATCTATAAGAACTTACA GATACAGCAGAACGTGATCGGAGAAAGTTTCCGGCATCAGGTGA AAAAACTGCTCTTTCTGGGCAGTACCTGCATCTATCCGAGGGATG CCGAACAGCCGATGAAAGAGGACGTCTTGCTCACTTCTCCACTG GAATATACCAATGAACCTTATGCCATAGCCAAAATAGCCGGACTG AAAATGTGCGAAAGCTTCAACCTGCAATATGGAACGAACTACATC GCCGTGATGCCGACCAACCTGTATGGGCCGAACGATAACTTTGA |
| | CTTGGAACGCAGTCATGTGCTGCCTGCCATGATCCGTAAAGTTCA CTTGGCACACTGCCTGAAAAAAGGAGATTGGGAGGCCGTGCGTA AAGATATGAACCTGCGTCCGGTAGAAGGCATCAGTGGTGCCAAC TCCAACGAAGAGATCCTCCGGATTCTCCGGAAATACGGCATTACT GAAACGGAGGTGACACTTTGGGGAACAGGAACGCCTCTAAGGGA ATTTCTTTGGAGTGAAGAAATGGCAGATGCCAGTGTCTTCGTAAT GGAACATGTGGACTTCAAGGATACCTACAAAACCGGTGCAAAAGA CATCCGCAACTGCCACATCAATATAGGTACCGGCAAAGAAATTAC AATCCGCGAACTGGCCGGACTGATTGTAAATACAGTCGGCTATCA GGGTGAACTGACTTTTGACAGCAGTAAACCGGACGGAACCATGC GAAAACTTACCGATCCGTCGAAATTGCACAACCTCGGATGGCATC ATAAGATCGATATTGAAGAGGGGGTACAGAAAATGTACGAGTGGT ATCTGGGATAA |
| SEQ ID NO 7: FucT from H. *pylori* (HpFucT) (corresponding to NCBI-ProteinID AAD29863.1, and UniProt Q9X435) | ATGGCTTTTAAGGTGGTGCAAATTTGCGGGGGGCTTGGGAATCA AATGTTTCAATACGCTTTCGCTAAAAGTTTGCAAAAACACTCTAAT ACGCCTGTGCTGTTAGATATTACTTCTTTTGATTGGAGTAATAGAA AAATGCAATTAGAACTTTTCCCTATTGATTTGCCCTATGCGAGTGA AAAAGAAATCGCTATAGCTAAAATGCAACACCTCCCCAAGCTAGT AAGAAATGTGCTCAAATGCATGGGGTTTGATAGGGTGAGTCAAGA AATCGTTTTTGAATACGAGCCTAAATTGTTAAAGACAAGCCGCTTG ACTTATTTTTATGGCTATTTTCAAGATCCACGATATTTTGATGCTAT ATCCCCTTTAATCAAGCAAACCTTCACCCTACCCCCCCCCCCCGA AAATGGAAATAATAAAAAAAAAGAGGAAGAATACCACCGCAAGCT TGCTTTGATTTTAGCCGCTAAAAACAGCGTGTTTGTGCATATAAGA AGAGGGGATTATGTGGGGATTGGCTGTCAGCTTGGTATTGATTAT CAAAAAAAGGCGCTTGAGTATATGGCAAAGCGCGTGCCAAACAT GGAGCTTTTTGTGTTTTGCGAAGACTTAGAATTCACGCAAAATCTT GATCTTGGCTACCCTTTTATGGACATGACCACTAGGGATAAAGAA GAAGAAGCGTATTGGGATATGCTGCTCATGCAATCTTGCAAGCAT GGCATTATCGCTAACAGCACTTATAGCTGGTGGGCGGCCTATTTG ATAAACAATCCAGAAAAAATCATTATTGGCCCCAAACACTGGCTTT TTGGGCATGAGAATATCCTTTGTAAGGAATGGGTGAAAATAGAAT CCCATTTTGAGGTGAAATCCCAAAAGTATAACGCTTAA |
| SEQ ID NO 8: Codon optimized FucT from *H*. *mustelae* (codon optimized HmFucT) (corresponding to NCBI-ProteinID CBG40460.1, and UniProt D3UIY5) | ATGGCACAGGTCgTGGACTTCAAGATCGTCCAGGTCCACGGCGG CCTCGGCAACCAGATGTTCCAGTACGCATTCGCAAAGAGCCTCC AGACCCACCTCAACATCCCGGTCCTCCTCGACACCACCTGGTTC GACTACGGCAACCGTGAGCTCGGCCTCCACCTCTTCCCGATCGA CCTCCAGTGCGCAAGCGCACAGCAGATCGCAGCAGCACACATGC AGAACCTCCCGCGTCTCGTCCGTGGCGCACTCCGTCGTATGGGC CTCGGCCGTGTCAGCAAGGAGATCGTCTTCGAGTACATGCCGGA GCTCTTCGAGCCGAGCCGTATCGCATACTTCCACGGCTACTTCCA GGACCCGCGTTACTTCGAGGACATCAGCCCGCTCATCAAGCAGA CCTTCACCCTCCCGCACCCGACCGAGCACGCAGAGCAGTACAGC CGTAAGCTCAGCCAGATCCTCGCAGCAAAGAACAGCGTCTTCGT CCACATCCGTCGTGGCGACTACATGCGTCTCGGCTGGCAGCTCG ACATCAGCTACCAGCTCCGTGCAATCGCATACATGGCAAAGCGT GTCCAGAACCTCGAGCTCTTCCTCTTCTGCGAGGACCTCGAGTTC GTCCAGAACCTCGACCTCGGCTACCCGTTCGTCGACATGACCAC CCGTGACGGCGCAGCACACTGGGACATGATGCTCATGCAGAGCT GCAAGCACGGCATCATCACCAACAGCACCTACAGCTGGTGGGCA GCATACCTCATCAAGAACCCGGAGAAGATCATCATCGGCCCGAG |
| | CCACTGGATCTACGGCAACGAGAACATCCTCTGCAAGGACTGGG TCAAGATCGAGAGCCAGTTCGAGACCAAGAGCTAA |
| SEQ ID NO 9: LacY from *E*. *coli* (LacY_EC) (corresponding to NCBI-ProteinID ADT73957, and UniProt E0J0Q8) | ATGTACTATTTAAAAAACACAAACTTTTGGATGTTCGGTTTATTCTT TTTCTTTTACTTTTTTATCATGGGAGCCTACTTCCCGTTTTCCCG ATTTGGCTACATGACATCAACCATATCAGCAAAAGTGATACGGGT ATTATTTTGCCGCTATTTCTCTGTTCTCGCTATTATTCCAACCGC TGTTTGGTCTGCTTTCTGACAAACTCGGGCTGCGCAAATACCTGC TGTGGATTATTACCGGCATGTTAGTGATGTTTGCGCCGTTCTTTAT TTTTATCTTCGGGCCACTGTTACAATACAACATTTTAGTAGGATCG ATTGTTGGTGGTATTTATCTAGGCTTTTGTTTTAACGCCGGTGCG CCAGCAGTAGAGGCATTTATTGAGAAAGTCAGCCGTCGCAGTAAT TTCGAATTTGGTCGCGCGCGGATGTTTGGCTGTGTTGGCTGGGC GCTGTGTGCCTCGATTGTCGGCATCATGTTCACCATCAATAATCA GTTTGTTTTCTGGCTGGGTTCTGGCTGTGCACTCATCCTCGCCGT TTTACTCTTTTTCGCCAAAACGGATGCGCCCTCTTCCGCCACGGT TGCCAATGCGGTAGGTGCCAACCATTCGGCATTTAGCCTTAAGCT GGCGCTGGAACTGTTCAGACAGCCAAAACTGTGGTTTTTGTCACT GTATGTTATTGGCGTTTCCTGCACCTACGATGTTTTTGACCAACA GTTTGCTAATTTCTTTACTTCGTTCTTTGCTACCGGTGAACAGGGT ACGCGGGTATTTGGCTACGTAACGACAATGGGCGAATTACTTAAC GCCTCGATTATGTTCTTTGCGCCACTGATCATTAATCGCATCGGT GGGAAAAACGCCCTGCTGCTGGCTGGCACTATTATGTCTGTACG TATTATTGGCTCATCGTTCGCCACCTCAGCGCTGGAAGTGGTTAT TCTGAAAACGCTGCATATGTTTGAAGTACCGTTCCTGCTGGTGGG CTGCTTTAAATATATTACCAGCCAGTTTGAAGTGCGTTTTTCAGCG ACGATTTATCTGGTCTGTTTCTGCTTCTTTAAGCAACTGGCGATGA TTTTTATGTCTGTACTGGCGGGCAATATGTATGAAAGCATCGGTTT CCAGGGCGCTTATCTGGTGCTGGGTCTGGTGGCGCTGGGCTTCA CCTTAATTTCCGTGTTCACGCTTAGCGGCCCCGGCCCGCTTTCTC TACTGCGTCGTCAGGTGAATGAAGTCGCTTAA |
| SEQ ID NO 10: LacY from *Lactobacillus delbrueckii* (LacY_Ldb) (corresponding to NCBI-ProteinID CAI98004, and UniProt P22733) | ATGAAGAAAAAGCTTGTCTCACGCTTGTCGTACGCGGCCGGTGC CTTTGGCAACGACGTCTTCTACGCGACTCTGTCAACCTACTTTAT CGTCTTCGTCACCACCCACCTCTTTAATGCCGGTGACCACAAGAT GATCTTTATCATCACCAACTTGATCACCGCCATCCGGATCGGGGA AGTCCTGCTCGACCCCTTGATCGGTAACGCCATCGACCGGACCG AAAGCCGGTGGGGGAAGTTCAAGCCCTGGGTTGTGGCGGGGG GATCATCAGCTCATTAGCCCTCTTAGCCCTCTTTACCGACTTTGG CGGCATTAACCAAAGCAAACCCGTTGTTTACTTAGTAATCTTCGG TATTGTTTACTTGATTATGGATATCTTCTACTCATTTAAAGACACTG GCTTCTGGGCCATGATCCCGGCCTTGTCCCTGGATTCCCGGGAA AGAGAGAAGACCTCCACCTTCGCCAGAGTCGGCTCCACCATCGG GGCCAACCTGGTCGGGGTAGTCATCACCCCAATCATCCTCTTCTT CTCGGCTAGCAAGGCCAACCCCAACGGGGATAAGCAGGGCTGG TTCTTCTTTGCCTTGATCGTGGCCATTGTCGGCATCTTGACCTCA ATTACCGTTGGTCTTGGTACTCACGAAGTAAAATCCGCCCTGCGG GAAAGCAATGAAAAGACCACTTTGAAGCAGGTCTTTAAGGTCCTG GGGCAAAACGACCAGCTCCTCTGGCTGGCCTTTGCCTACTGGTT TTACGGCCTGGGTATCAACACCCTGAACGCTCTGCAACTTTACTA CTTCTCATACATCTTAGGCGATGCCCGCGGCTACAGCCTGCTTTA CACCATCAACACCTTTGTCGGTTTAATCTCTGCATCCTTCTTCCCA TCACTGGCCAAGAAGTTCAACAGAAATCGCCTCTTCTACGCCTGC ATCGCGGTGATGCTGTTAGGGATCGGGGTCTTCTCCGTGGCCAG |
| | CGGTTCTCTGGCCCTGTCCCTTGTTGGGGCAGAATTCTTCTTTAT TCCGCAGCCTCTGGCCTTCCTGGTCGTTTTGATGATCATCTCTGA CGCTGTTGAATACGGCCAGCTGAAAACTGGCCACAGAGACGAAG CTTTGACCCTGTCTGTCCGGCCATTGGTCGATAAGCTGGGCGGG GCCTTGTCCAACTGGTTTGTTTCCTTGATTGCCTTAACTGCCGGC ATGACCACTGGGGCGACTGCCTCAACAATTACAGCTCATGGCCA GATGGTCTTCAAGTTAGCTATGTTTGCCTTACCGGCAGTCATGCT CTTGATCGCTGTTTCTATTTTCGCCAAAAAGGTCTTCTTGACTGAA GAAAAGCACGCGGAAATCGTCGACCAGCTGGAAACTCAATTCGG CCAAAGCCATGCCCAAAAGCCGGCGCAAGCTGAAAGCTTCACTT TGGCCAGCCCAGTCTCCGGACAATTAATGAACCTGGACATGGTT GACGACCCGGTCTTTGCCGACAAAAAGTTAGGCGACGGCTTTGC CCTGGTGCCAGCAGACGGTAAGGTCTACGCGCCATTTGCCGGTA CTGTCCGCCAGCTGGCCAAGACCCGGCACTCGATCGTCCTGGAA AATGAACATGGGGTCTTGGTCTTGATTCACCTTGGCCTGGGCAC GGCCAAATTAAACGGGACTGGCTTTGTCAGCTATGTTGAAGAGG GCAGCCAGGTAGAAGCCGGCCAGCAGATCCTGGAATTCTGGGAC CCGGCGATCAAGCAGGCCAAGCTGGACGACACGGTAATCGTGAC CGTCATCAACAGCGAAACTTTCGCAAATAGCCAGATGCTCTTGCC GATCGGCCACAGCGTCCAAGCCCTGGATGATGTATTCAAGTTAG AAGGGAAGAATTAG |
| SEQ ID NO 11: Lacl from *Escherichia Coli* (corresponding to NCBI-ProteinID ADT73959, and UniProt A0A0H3EV00) | TCACTGCCCGCTTTCCAGTCGGGAAACCTGTCGTGCCAGCTGCA TTAATGAATCGGCCAACGCGCGGGGAGAGGCGGTTTGCGTATTG GGCGCCAGGGTGGTTTTTCTTTTCACCAGTGAGACGGGCAACAG CTGATTGCCCTTCACCGCCTGGCCCTGAGAGAGTTGCAGCAAGC GGTCCACGCTGGTTTGCCCCAGCAGGCGAAAATCCTGTTTGATG GTGGTTAACGGCGGGATATAACACGAGCTGTCTTCGGTATCGTC GTATCCCACTACCGAGATATCCGCACCAACGCGCAGCCCGGACT CGGTAATGGCGCGCATTGCGCCCAGCGCCATCTGATCGTTGGCA ACCAGCATCGCAGTGGGAACGATGCCCTCATTCAGCATTTGCAT GGTTTGTTGAAAACCGGACATGGCACTCCAGTCGCCTTCCCGTTC CGCTATCGGCTGAATTTGATTGCGAGTGAGATATTTATGCCAGCC CGCCAGACGCAGACGCGCCGAGACAGAACTTAATGGGCCCGCT AACAGCGCGATTTGCTGGTGACCCAATGCGACCAGATGCTCCAC GCCCAGTCGCGTACCGTCTTCATGGGAGAAAATAATACTGTTGAT GGGAGTCTGGTCAGAGACATCAAGAAATAAAGCAGGAACATTAG CGCAGGCTGCTTCCACAGCAATGGCATCCTGGTCATCCAGCGGA TAGTTAATGATCAGCCCACTGACGCGTTGCGCGAGAAGGTTGTG CACCGCCGCTTTACAGGCTTCGACGCCGCTTCGTTCTACCATCG ACACCACCACGCTGGCACCCAGTTGATCGGCGCGAGATTTAATC GCCGCGACAATTTGCGACGGCGCGTGCAGGGCCAGACTGGAGG TGGCAACGCCAATCAGCAACGACTGTTTGCCCGCCAGTTGTTGT GCCACGCGGTTGGGAATGTAATTCAGCTCCGCCATCGCCGCTTC CACTTTTTCCCGCGTTTTCGCAGAAACGTGGCTGGCCTGGTTCAC CACGCGGGAAACGGTCTGATAAGAGACACCGGCATACTCTGCGA CATCGTATAGCGTTACTGGTTTCACATTCACCAC |

The invention therefore encompasses a genetically modified corynebacterium as described herein comprising an exogenous nucleic acid sequence selected from the group consisting of:
a) a nucleic acid sequence comprising a nucleotide sequence that encodes an enzyme/protein encoded by a nucleotide sequence according to SEQ ID NO 1, 2, 3, 4, 5, 6, 7, 8, 9, 10 or 11;
b) a nucleic acid sequence which is complementary to a nucleotide sequence in accordance with a);
c) a nucleic acid sequence comprising a nucleotide sequence having sufficient sequence identity to be functionally analogous/equivalent to a nucleotide sequence according to a) or b), comprising preferably a sequence identity to a nucleotide sequence according to a) or b) of at least 70%, 80%, preferably 90%, more preferably 95%;
d) a nucleic acid sequence which, because of the genetic code, is degenerated into a nucleotide sequence according to a) through c); and/or
e) a nucleic acid sequence according to a nucleotide sequence of a) through d) which is modified by deletions, additions, substitutions, translocations, inversions and/or insertions and functionally analogous/equivalent to a nucleotide sequence according to a) through d).

Functionally analogous sequences refer to the ability to encode a functional gene product. Functionally analogous sequences refer to the ability to encode a functional gene product and to enable the same or similar functional effect as the gene products of the disclosed sequence.

Function of the proteins/enzyme can be determined by suitable tests for the activity of the respective enzyme/protein, which are routine tests for a skilled person. Appropriate assays for determining enzymatic activity are known in the art.

As used herein, "nucleic acid" shall mean any nucleic acid molecule, including, without limitation, DNA, RNA and hybrids or modified variants thereof. An "exogenous nucleic acid" or "exogenous genetic element" relates to any nucleic acid or nucleic acid sequence introduced into the bacterial cell, which is not a component of the cells "original" or "natural" genome. Exogenous nucleic acids may be integrated or non-integrated, or relate to stably transfected/transformed nucleic acids, and can be an integrative or episomal vector/plasmid containing the respective gene of interest.

In preferred embodiments, an exogenous nucleic acid sequence is chromosomally integrated. The term "chromosomal integration" relates to integration of the target genes into the host chromosome, which is a preferable strategy to overcome the drawbacks of plasmid-based overexpression. In bacteria such as for example *Escherichia coli,* homologous recombination, site-specific recombination and transposon-mediated gene transposition are often used to achieve chromosomal integration.

As used herein, the term expression cassette relates to a distinct component of a nucleic acid sequence, which can for example be comprised by a vector/plasmid DNA or which can be integrated into a host genome. An expression cassette comprises a gene/a coding sequence and regulatory sequence to enable expression of the gene/coding sequence in the genetically modified cell/bacterium. The expression cassette directs the cell's machinery to make RNA and protein(s). An expression cassette can comprise of one or more genes and the sequences controlling their expression. An expression cassette usually comprises three components: a promoter sequence, an open reading frame, and a 3' untranslated region that. Different expression cassettes can be transfected into different organisms including bacteria, yeast, plants, and mammalian cells as long as the correct regulatory sequences are used.

In embodiments, two or more of the transgenes can be comprised/encoded by a continuous exogenous nucleic acid sequence comprising coding sequences of the two or more transgenes, for example within a single operon. An operon is a functioning unit of DNA containing a cluster of genes, e.g. at least two genes, under the control of a single promoter. The genes are transcribed together into an mRNA strand and either translated together in the cytoplasm or undergo splicing to create monocistronic mRNAs that are translated separately, i.e. several strands of mRNA that each encode a single or multiple gene products. The result of this is that the genes contained in the operon are either expressed together or not at all. Several genes must be co-transcribed to define an operon. Originally, operons were thought to exist solely in prokaryotes (which includes organelles like plastids that are derived from bacteria), but since the discovery of the first operons in eukaryotes in the early 1990s, more evidence has arisen to suggest they are more common than previously assumed. In general, expression of prokaryotic operons leads to the generation of polycistronic mRNAs, while eukaryotic operons lead to monocistronic mRNAs. Operons are also found in viruses such as bacteriophages. For example, T7 phages have two operons. The first operon codes for various products, including a special T7 RNA polymerase which can bind to and transcribe the second operon. The second operon includes a lysis gene meant to cause the host cell to burst.

An operon is made up of several structural genes arranged under a common promoter and regulated by a common operator. It is defined as a set of adjacent structural genes, plus the adjacent regulatory signals that affect transcription of the structural genes. The regulators of a given operon, including repressors, corepressors, and activators, are not necessarily coded for by that operon. The location and condition of the regulators, promoter, operator and structural DNA sequences can determine the effects of common mutations. An operon contains one or more structural genes which are generally transcribed into one polycistronic mRNA (a single mRNA molecule that codes for more than one protein). However, the definition of an operon does not require the mRNA to be polycistronic, though in practice, it usually is. Upstream of the structural genes lies a promoter sequence which provides a site for RNA polymerase to bind and initiate transcription. Close to the promoter lies a section of DNA called an operator.

An operon is made up of 3 basic DNA components: (i.) Promoter, which is a nucleotide sequence that enables a gene to be transcribed. The promoter is recognized by RNA polymerase, which then initiates transcription. In RNA synthesis, promoters indicate which genes should be used for messenger RNA creation - and, by extension, control which proteins the cell produces. (ii.) Operator - a segment of DNA to which a repressor bind. It is classically defined in the lac operon as a segment between the promoter and the genes of the operon. The main operator (O1) in the lac operon is located slightly downstream of the promoter; two additional operators, O1 and 03 are located at -82 and +412, respectively. In the case of a repressor, the repressor protein physically obstructs the RNA polymerase from transcribing the genes. (iii.) Structural genes - the genes that are co-regulated by the operon.

Not always included within the operon, but important in its function is a regulatory gene, a constantly expressed gene which codes for repressor proteins. The regulatory gene does not need to be in, adjacent to, or even near the operon to control it. An inducer (small molecule) can displace a repressor (protein) from the operator site (DNA), resulting in an uninhibited operon. Alternatively, a corepressor can bind to the repressor to allow its binding to the operator site. A good example of this type of regulation is seen for the trp operon.

Suitable promoters for the transgenes of the invention are disclosed herein and include constitutive promotors such as Tuf, GlyA, GroeL, GroES, DnaK, GapA, SOD, PGK and inducible promotors such as Tac, Lac and Trc promoters or synthetic promotors.

For control of expression of the respective enzymes to be expressed in *corynebacteria* of the invention, constitutive and inducible promoters can be used. As used herein "inducible expression" or "conditional expression" relates to a state, multiple states or system of gene expression, wherein the gene of interest, is preferably not expressed, or in some embodiments expressed at negligible or relatively low levels, unless there is the presence of one or more molecules (an inducer) or other set of conditions in the cell, preferably the *corynebacterium,* that allows for gene expression. Inducible promoters may relate to either naturally occurring promoters that are expressed at a relatively higher level under certain biological conditions, or to other promoters comprising any given inducible element. Inducible promoters may refer to those induced by external factors, for example by administration of a small drug molecule or other externally applied signal. An example of such a small molecule used for inducible expression is isopropyl β-d-1-thiogalactopyranoside (IPTG), which is a molecular biology reagent. This compound is a molecular mimic of allolactose, a lactose metabolite that triggers transcription of the lac operon, and it is therefore used to induce protein expression where the gene is under the control of the lac operator. Examples Positively regulated bacterial expression systems have been reviewed by Brautaset et al (Microbial Biotechnology (2009)2(1), 15-30).

A transcription terminator is a section of nucleic acid sequence that marks the end of a gene or operon in a DNA sequence during transcription. This sequence mediates transcriptional termination by providing signals in the newly synthesized transcript RNA that trigger processes which release the transcript RNA from the transcriptional complex. These processes include the direct interaction of the mRNA secondary structure with the complex and/or the indirect activities of recruited termination factors. Release of the transcriptional complex frees RNA polymerase and related transcriptional machinery to begin transcription of new mRNAs. Suitable examples of terminator sequences that can be used in the context of the invention include any rho-independent terminators such as T7 terminator and a rrnB terminator.

In embodiments, the genetically modified *corynebacterium* of the invention is defective for functional expression of one or more glycosyltransferases involved in *corynebacterial* cell wall biosynthesis.

Almost all *Corynebacterium* species including *corynebacterium glutamicum* are characterized by a complex cell wall architecture: the plasma membrane of these bacteria is covered by a peptidoglycan layer, which itself is covalently linked to arabinogalactan, an additional heteropolysaccharide meshwork. Bound to this, an outer layer of mycolic acids is found which is functionally equivalent to the outer membrane of Gram-negative bacteria. As top layer, outer surface material composed of free polysaccharides, glycolipids, and proteins (including S-layer proteins, pili, and other surface proteins) is found. The general structure and composition of the *corynebacterial* cell envelope were earlier reviewed by M. Daffé (Handbook of Corynebacterium glutamicum, L. Eggeling and M. Bott, Eds., pp. 121-148, Taylor & Francis, Boca Raton, Fla, USA, 2005) and L. Eggeling et al. (Corynebacteria, A. Burkovski, Ed., pp. 267-294, Caister Academic Press, Norfolk, UK, 2008).

Glycosyltransferases are (GTFs, Gtfs) are enzymes that establish natural glycosidic linkages. They catalyze the transfer of saccharide moieties from an activated nucleotide sugar (also known as the "glycosyl donor") to a nucleophilic glycosyl acceptor molecule, the nucleophile of which can be oxygen- carbon-, nitrogen-, or sulfur-based. The result of glycosyl transfer can be a carbohydrate, glycoside, oligosaccharide, or a polysaccharide. Some glycosyltransferases catalyse transfer to inorganic phosphate or water. Glycosyl transfer can also occur to protein residues, usually to tyrosine, serine, or threonine to give O-linked glycoproteins, or to asparagine to give N-linked glycoproteins. Mannosyl groups may be transferred to tryptophan to generate C-mannosyl tryptophan, which is relatively abundant in eukaryotes. Transferases may also use lipids as an acceptor, forming glycolipids, and even use lipid-linked sugar phosphate donors, such as dolichol phosphates.

Glycosyltransferases (GTs) are known to be critically involved in the bacterial cell wall biosynthesis, in particular in *Corynebacterium.* In particular, GTs are involved in the construction of the peptidoglycan layer of the *Corynebacterium* cell wall. Peptidoglycan is an essential polymer that forms a protective shell around bacterial cell membranes. The peptidoglycan layer surrounds the cytoplasmic membrane of *Corynebacteria* and functions as an exoskeleton, maintaining cell shape and stabilizing the membrane against fluctuations in osmotic pressure. Peptidoglycan is synthesized in an intracellular phase in which UDP-N-acetylglucosamine is converted to a diphospholipid-linked disaccharide-pentapeptide known as Lipid II, and in an extracellular phase in which the disaccharide (NAG-NAM) subunits of translocated Lipid II are coupled by peptidoglycan glycosyltransferases (PGTs; also known as transglycosylases) to form linear carbohydrate chains, which are cross-linked through the attached peptide moieties by transpeptidases. PGTs are defined by the presence of five conserved sequence motifs and exist in two forms: (i) as N-terminal glycosyltransferase domains in bifunctional proteins that also contain a C-terminal transpeptidase domain [called class A penicillin-binding proteins (PBPs)], and (ii) as monofunctional proteins (MGTs) that do not contain transpeptidase domains. Different bacteria typically contain different numbers and types of PGTs, and it is thought that the different PGTs play different roles during the bacterial cell cycle, with some involved primarily in cell elongation and others recruited to the septal region during cell division. Regardless of their cellular roles, all PGTs catalyze glycosyltransfer from a polyprenyl-diphosphate moiety on the anomeric center of an N-acetyl muramic acid (NAM) unit to the C4 hydroxyl of an N-acetylglucosamine (NAG) moiety. The mechanism of glycosyltransfer is not well understood, and it is taking considerable effort to identify soluble, well behaved PGT domains to use as model systems for detailed mechanistic and structural analysis.

In the context of the present invention, it was surprisingly found that *Corynebacteria* and in particular *Corynebacterium glutamicum* with deficiency for one of more GTs leads to a markedly reduced foam production during the culturing process and also increases the yield of biomolecules produced by the bacteria, such as fucosyllactose.

In embodiments, the *Corynebacterium glutamicum* is defective for functional expression of one or more glycosyltransferases, preferably selected from the group comprising GTs comprising cgp_3313 (MrcB; GT51), cgp_0336 (PonA; GT51); cgp_3166 (GT4); cgp_2400 (GT4); cgp_1876 (GT4); cgp_1268 (GlgA; GT4); cgp_0554 (GT4); cgp_3191 (GlfT; GT2); cgp_1672 (PpmC; GT2); cgp_1180 (GT2); cgp_0848 (WbbL; GT2); cgp_0730 (GT2); cgp_0396 (GT2); cgp_0394 (GT2); cgp_0246 (GT2); cgp_0163 (GT2); cgp_2393 (GT87,GT87); cgp_2390 (GT87); cgp_2389 (GT87); cgp_2385 (GT87); and cgp_3164.

In embodiments, the present invention relates to a method of producing biomolecules, such as fucosyllactose, the method comprising culturing a genetically modified *corynebacterium* of the present invention in a medium supplemented with lactose. In such methods, the bacteria can be cultivated, for example, in shake flask cultivation or Fedbatch cultivation, as is well established in the art. The method of cultivating the bacteria of the invention for the production of biomolecules can be performed according to well established protocols of the state of the art, which are known to the skilled person and which are disclose in the literature, for example in the Handbook of Corynebacterium glutamicum (Edited By Lothar Eggeling, Michael Bott; doi.org/10.1201/9781420039696) or in Corynebacterium Glutamicum: Biology and Biotechnology (edited by Tatsumi, Nami, Inui, Masayuki; Springer; 2013. Edition (14. August 2012)).

In embodiments, the bacteria of the invention are cultured in a fed-batch process. Fed-batch culture is, in the broadest sense, defined as an operational technique in biotechnological processes where one or more nutrients (substrates) are fed (supplied) to the bioreactor during cultivation and in which the product(s) remain in the bioreactor until the end of the run. An alternative description of the method is that of a culture in which a base medium supports initial cell culture and a feed medium is added to prevent nutrient depletion. It is also a type of semi-batch culture. In some cases, all the nutrients are fed into the bioreactor. The advantage of the fed-batch culture is that one can control concentration of fed-substrate in the culture liquid at arbitrarily desired levels (in many cases, at low levels). Generally speaking, fed-batch culture is superior to conventional batch culture when controlling concentrations of a nutrient (or nutrients) affects the yield or productivity of the desired metabolite.

In the context of the invention, fed-batch culture can be performed as described in this paragraph, representing a preferred embodiment. The Fed-batch culture can be performed in a 1-L fermenter (Multifors, Infors) with 0.4 L working volume. The seed cultures may be prepared in CGXII medium in shake flasks as described herein; the fermenter can be inoculated to an OD600 of 1 in CGXII medium with 4% w/v glucose. The agitation and aeration are preferably set at 800 rpm and 1vvm, respectively. The pH is maintained at 7.0 and temperature at 30 °C throughout the fermentation. Dissolved oxygen (DO) is measured by an electrode, 100% is set by oxygen-saturated distilled water. 1 mM IPTG is added at t = 16 h and 4 g/L lactose is added at t = 25 h. Feeding medium contained 400 g/L glucose and 100 g/L (NH4)2SO4, and the feed frequency is variably adjusted automatically depending on the DO signal. The feeding starts when DO exceeds 30% and stops when DO felt again under the set-point.

In preferred embodiments of the methods of the invention, the *Corynebacteria* are cultured in the presence of lactose and glucose in order to provide the substrates for the fucosyllactose biosynthesis pathway comprised by bacteria of the invention.

In embodiments of the method of the invention, the bacteria of the invention are cultured in a shake flask cultivation. In such embodiments, all shake flasks cultivations can be performed at 30 °C at agitation speed of 200 rpm. Cells cultured on BHIS agar plates for 2 days are sequentially transferred into 10 ml of the LBG medium (yeast extract: 5 g/L, peptone: 10 g/L, NaCl: 10 g/L, glucose: 1 g/L) grown overnight, and then to a 500 mL flask containing 150 mL of CGXII medium (20 g/L (NH₄)₂SO₄, 5 g/L urea, 1 g/L KH₂PO₄, 1 g/L K₂HPO₄, 0.25 g/L MgSO₄·7H₂O, 10 mg/L CaCI2, 10 mg/L FeSO₄·7H₂O, 10 mg/L MnSO₄·H₂O, 1 mg/L ZnSO₄·7H₂O, 0.2 mg/L CuSO₄·5H₂O, 0.02 mg/L NiCl₂·6H₂O, 0.2 mg/L biotin, 30 mg/L 3,4-dihydroxybenzoic acid, and 21 g/L 3-morpholinopropanesulfonic acid (MOPS); pH 7.0) with 4% w/v glucose. 1 mM isopropyl-β-D-thiogalactoside (IPTG) was added after 4 h, additionally, 1 g/L Lactose was supplemented to the strains after 8 h of inoculation.

### FIGURES

The invention is further described by the following figures. These are not intended to limit the scope of the invention but represent preferred embodiments of aspects of the invention provided for greater illustration of the invention described herein.

### Description of the figures:

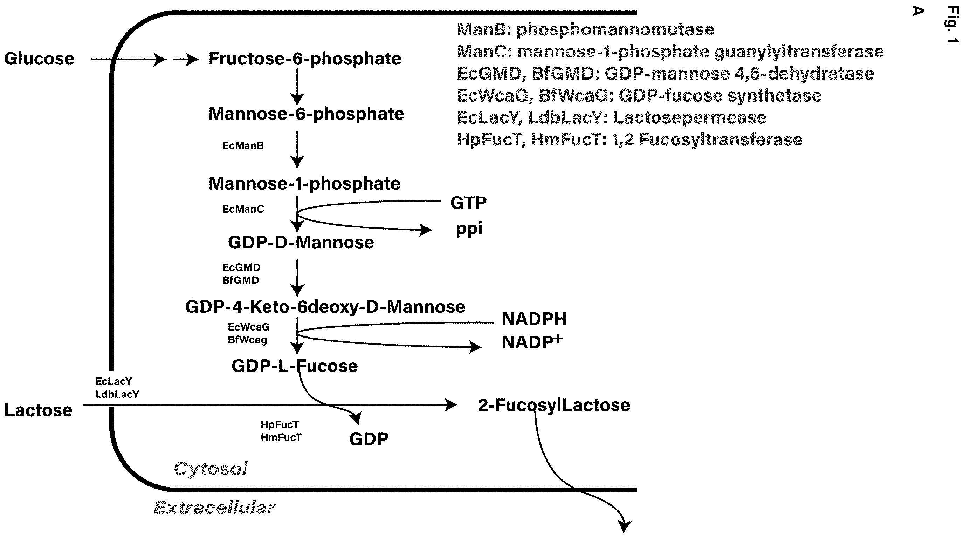

**Figure 1****:** Metabolic pathway representing the biosynthesis of 2-FucosylLactose engineered in C. *glutamicum.* **A.** *C. glutamicum* has no lactose transport capabilities, hence genes for Lactose transporters in addition to pathway enzymes are overexpressed under regulated and/or unregulated promotors to enhance the production of 2FL from glucose. (Ec = E. coli; Bf = *Bacteroides fragilis;* Ldb = *Lactobacillus delbrueckii;* Hp = *Helicobater pylori*).

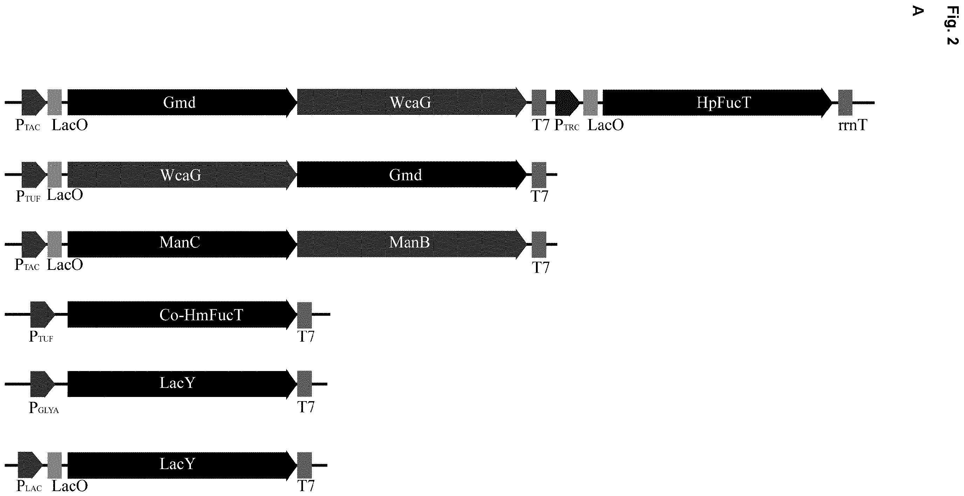

**Figure 2****:** Schematic representation of gene cassettes of exogenous nucleic acid sequences of the invention for chromosomal integration in the genetically modified *corynebacteria* of the invention.

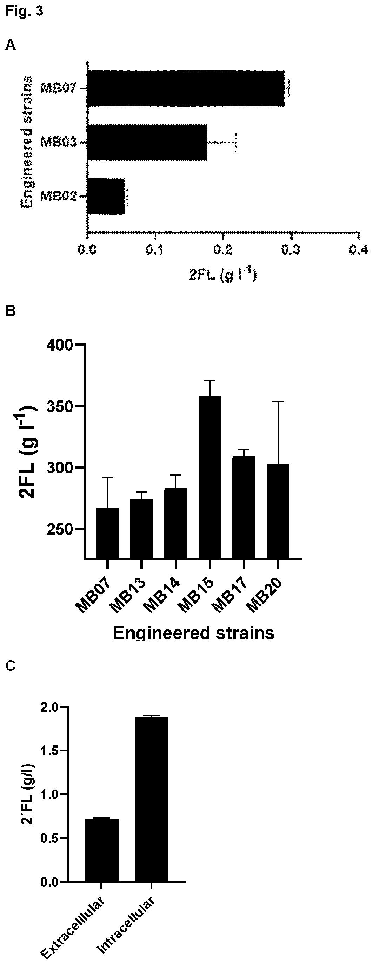

**Figure 3**: Rational engineering of 2'-Fucosyllactose (2'-FL) production pathway in *C*. *glutamicum.* 2FL produced into culture supernatant by different *C glutamicum* strains after 72 h of shake flask cultivation in defined CGXII medium with 4 % (w/v) glucose. All data were from biological triplicates, and error bars represent standard deviation. **A.** Comparison of different genetically engineered strains of the invention. **B**. Effect of deletion of different glycosyltransferases (MB13, MB14, MB15, MB17, MB20) compared to strain MB07. **C**. Intra and extracellular (into media) accumulation of 2'-FL in MB04 strain at the end of fedbatch fermentation in defined CGXII medium with 4 % (w/v) glucose and 4 g/L lactose. Feeding medium contained 400 g/L glucose and 100 g/L (NH4)₂SO₄, and the feed frequency was variably adjusted automatically depending on the DO signal. The feeding started when DO exceeds 30% and stops when DO felt again under the set-point. Samples collected were analyzed for 2FL production by HPLC.

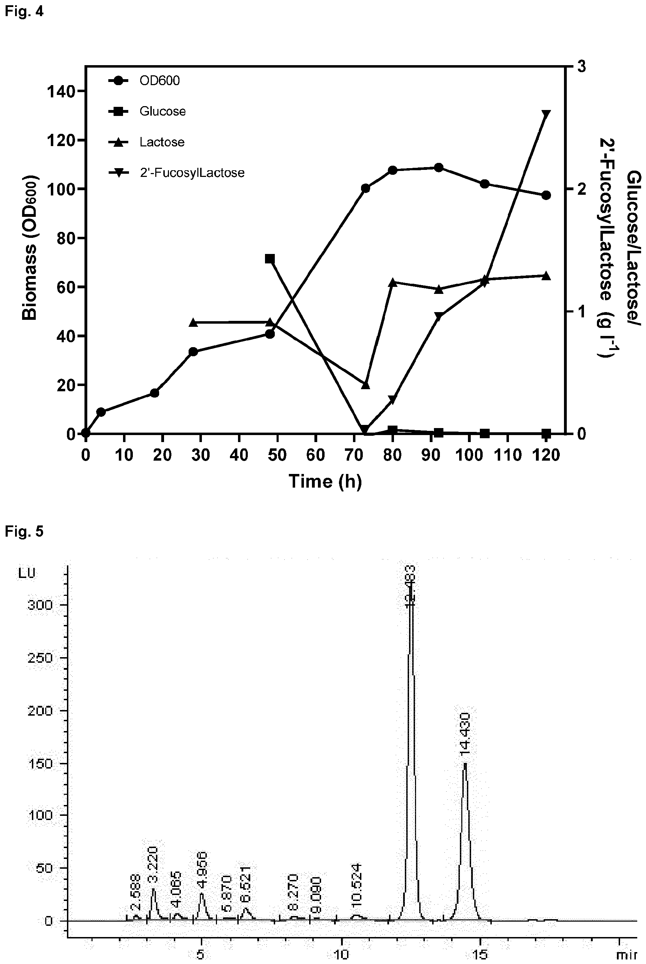

**Figure 4****:** *Fedbatch fermentation.* Fed-batch culture was performed with MB15 in a 1-L fermenter (Multifors, Infors) with 0.4 L working volume. The seed cultures were prepared in CGXII medium in shake flasks as described earlier; the fermenter was inoculated to an OD₆₀₀ of 1 in CGXII medium with 4% w/v glucose. The agitation and aeration were set at 800 rpm and 1vvm, respectively. The pH was maintained at 7.0 and temperature at 30 °C throughout the fermentation. Dissolved oxygen (DO) was measured by an electrode, 100% was set by oxygen-saturated distilled water. 1 mM IPTG was added at t = 16 h and 4 g/L lactose was added at t = 25 h. Feeding medium contained 400 g/L glucose and 100 g/L (NH4)₂SO₄, and the feed frequency was variably adjusted automatically depending on the DO signal. The feeding started when DO exceeds 30% and stops when DO felt again under the set-point. Samples collected were analyzed for 2FL production by HPLC.
**Figure 5****:** Determination of extracellularly produced 2'-fucosyllactose (2-FL). 2-amino benzamide labeled 2'-fucosyllactose (2-FL) from media after Fed-batch fermentation of the engineered Corynebacterium glutamicum strain MB07 analyzed by HILIC-HPLC. HILIC-HPLC was performed using a TSK-Gel Amide-80 4.6 x 250 mm column with a linear gradient of solvent A (100% ACN) and solvent B (0.1 % phosphoric acid) on an Agilent 1200 equipped with fluorescence detector. Fluorescence was measured at 420 nm with excitation at 320 nm (band width 16 nm).

### EXAMPLES

The invention is further described by the following examples. These are not intended to limit the scope of the invention but represent preferred embodiments of aspects of the invention provided for greater illustration of the invention described herein.

### Methods employed in the Examples

### Genetically engineered C. glutamicum strains generated and used in the examples.

The following strains of *C*. *glutamicum* were used in the present examples (see Table 2). The strain MB001 was purchased from DSMZ, while the other strains representing examples of the invention have been generated as explained below.

**Table 2. Strains used in the examples.**

| **strain** | **Relevant characteristics** | |
|---|---|---|
| MB001 | ATCC 13032 with Prophages - CGP1, CGP2, and CGP3 deleted | Baumgart, M. et al. (2013). |
| MB02 | MB001 - Δ*cgp_2725* ::P *Tac*-GMD_WcaG- P *Trc*-HpFucT, Δ*cgp_1213* ::P *Tac*-ManB_ManC, Δ*cgp_2854* ::P *GlyA*-LdbLacY, Δ*cgp_3151* ::P *Laciq*-Lacl; with integration of single copy genes | This work |
| | under regulated promotors - ManC_ManB GMD_WcaG operons from *Escherichia coli* under IPTG inducible Tac promotor, fucosyltransferase HpFucT gene from *Helicobacter pylori* under IPTG inducible Trc promotor, Lactose permease gene LdbLacY from *Lactobacillus delbrueckii* under growth phase regulated corynebacterial GlyA promotor and Lactose repressor gene Lacl under Laclq promotor) | |
| MB03 | MB02 Δcgp_1178::P Tuf-CO-HmFucT; codon optimized FucT from *Helicobacter mustulae* driven under strong constitutive promotor Tuf integrated in MB02. | This work |
| MB04 | MB03 Δ*cgp_1782* ::P *Tuf*-LacO-WcaG-GMD; genes WcaG and GMD from *Bacteriodes Fraglis* under constitutive promotor under Lac operator integrated in MB03. | This work |
| MB07 | MB04 Δ*cgp_2600::P LacUV5-LacY;* LacY gene from *Escherichia coli* under unregulated promotor integrated in MB04. | This work |
| MB13 | MB07 Δ*cgp_0336; deletion of gene in MB07 involved in the synthesis of peptidoglycan layer of cell wall.* | This study |
| MB14 | MB07 Δ*cgp_3191, deletion of gene in MB07 involved in the synthesis of plasma membrane of cell wall.* | |
| MB15 | MB07 Δ*cgp_3164; deletion of gene in MB07 involved in the synthesis of plasma membrane of cell wall.* | This study |
| MB17 | MB07 Δ*cgp_0554; deletion of gene in MB07 involved in the synthesis of outer membrane glycolipids of cell wall.* | This study |
| MB20 | MB07 Δ*cgp_2385; deletion of gene in MB07 involved in the synthesis of outer membrane glycolipids of cell wall.* | This study |

The transgenes of ***Table 3*** have been used for constructing the strains of ***Table 2.***

**Table 3. Genes used in transgenic strains of the invention.**

| Genes | NCBI-ProteinID | UniProt |
|---|---|---|
| ManB | ADT75633 | E0J145 |
| ManC | ADT75634 | E8PZ98 |
| GMD_EC | ADT75653 | E0J125 |
| WcaG_EC | ADT75652 | E0J126 |
| GMD, BF | CAH07586 | Q5LE66 |
| WcaG_BF | CAH07585 | Q5LE67 |
| Lactose Permease_EC | ADT73957 | E0J0Q8 |
| Lactose Permease_LDB | CAI98004 | P22733 |
| HPfucT | AAD29863.1 | Q9X435 |
| HMfucT | CBG40460.1 | D3UIY5 |

The genetic modification of the strains was carried out using the following protocol:
Chromosomal modifications are carried out by homologous recombination. Expression cassettes to be integrated or genes to be deleted were cloned together with about 1000 bp up- and downstream of chromosomal regions into vector suicide vector pK19mobsacB bearing kanamycin gene for antibiotic selection and a lethal gene levansucrase from *Bacillus subtilis.* Following transformation, clones with integrated vector were selected on BHIS agar plates with 25 µg/ml kanamycin in the first step. Selected clones grown in 3 ml BHIS at 30°C, 200 rpm overnight were plated at different dilutions on LB agar plates with 10% (w/v) sucrose, LB agar plates with 10% (w/v) sucrose and 25 µg/ml Kanamycin and LB agar plates with 25 µg/ml Kanamycin. Clones obtained on sucrose-plates were further checked for expression cassettes integration or deletion by PCR.

### Shake flask cultivation.

All shake flasks cultivations were performed at 30 °C under agitation speed of 200 rpm. Cells cultured on BHIS agar plates for 2 days were sequentially transferred into 10 ml of the LBG medium (yeast extract: 5 g/L, peptone: 10 g/L, NaCl: 10 g/L, glucose: 1 g/L) grown overnight, and then to a 500 mL flask containing 150 mL of CGXII medium (20 g/L (NH₄)₂SO₄, 5 g/L urea, 1 g/L KH₂PO₄, 1 g/L K₂HPO₄, 0.25 g/L MgSO₄·7H₂O, 10 mg/L CaCI2, 10 mg/L FeSO₄·7H₂O, 10 mg/L MnSO₄·H₂O, 1 mg/L ZnSO₄·7H₂O, 0.2 mg/L CuSO₄·5H₂O, 0.02 mg/L NiCl₂·6H₂O, 0.2 mg/L biotin, 30 mg/L 3,4-dihydroxybenzoic acid, and 21 g/L 3-morpholinopropanesulfonic acid (MOPS); pH 7.0) with 4% w/v glucose. 1 mM isopropyl-β-D-thiogalactoside (IPTG) was added after 4 h, additionally, 1 g/L Lactose was supplemented to the strains after 8 h of inoculation.

### Fedbatch cultivation

Fed-batch culture was performed in a 1-L fermenter (Multifors, Infors) with 0.4 L working volume. The seed cultures were prepared in CGXII medium in shake flasks as described earlier; the fermenter was inoculated to an OD₆₀₀ of 1 in CGXII medium with 4% w/v glucose. The agitation and aeration were set at 800 rpm and 1vvm, respectively. The pH was maintained at 7.0 and temperature at 30 °C throughout the fermentation. Dissolved oxygen (DO) was measured by an electrode, 100% was set by oxygen-saturated distilled water. 1 mM IPTG was added at t = 16 h and 4 g/L lactose was added at t = 25 h. Feeding medium contained 400 g/L glucose and 100 g/L (NH4)₂SO₄, and the feed frequency was variably adjusted automatically depending on the DO signal. The feeding started when DO exceeds 30% and stops when DO felt again under the set-point.

## Claims

1. Genetically modified (or engineered) corynebacterium for production of fucosyllactose,
**characterized in that** the corynebacterium is defective for functional expression of one or more glycosyltransferases involved in corynebacterial cell wall biosynthesis.

2. Genetically modified corynebacterium according to claim 1, wherein the one or more glycosyltransferases is selected from the group comprising cgp_3313 (MrcB; GT51), cgp_0336 (PonA; GT51); cgp_3166 (GT4); cgp_2400 (GT4); cgp_1876 (GT4); cgp_1268 (GlgA; GT4); cgp_0554 (GT4); cgp_3191 (GlfT; GT2); cgp_1672 (PpmC; GT2); cgp_1180 (GT2); cgp_0848 (WbbL; GT2); cgp_0730 (GT2); cgp_0396 (GT2); cgp_0394 (GT2); cgp_0246 (GT2); cgp_0163 (GT2); cgp_2393 (GT87,GT87); cgp_2390 (GT87); cgp_2389 (GT87); cgp_2385 (GT87); and cgp_3164.

3. The genetically modified corynebacterium according to any one of the preceding claims, wherein the corynebacterium is *corynebacterium glutamicum.*

4. The genetically modified corynebacterium according to the preceding claim, wherein the corynebacterium has been modified to express a permease for lactose import (preferably lactose permease (LacY)), GDP-D-mannose-4,6-dehydratase (GMD), GDP-L-fucose synthase (WcaG) and fucosyltransferase (FucT) from exogenous nucleic acid sequences.

5. The genetically modified corynebacterium according to claim 4, wherein the exogenous nucleic acid sequences encoding LacY, GMD, WcaG and FucT are chromosomally integrated.

6. The genetically modified corynebacterium according to any one of claims 4 or 5, wherein the corynebacterium additionally comprise exogenous nucleic acids for expression of phosphomannomutase (ManB) and GTP-mannose-1-phosphate guanylyltransferase (ManC), which are preferably chromosomally integrated.

7. The genetically modified corynebacterium according to any one of the preceding claims, where the *corynebacterium* exports fucosyllactose.


SEQUENCE LISTING

<110> GALAB Laboratories GmbH

<120> Glucosyltransferase deficient corynebacterium for the prodcution of
fucosyllactose

<130> 2397/20EP

<160> 11

<170> BiSSAP 1.3.6

<210> 1
<211> 1371
<212> DNA
<213> Bacteria <prokaryote>


<220>
<223> ManB (corresponding to NCBI-ProteinID ADT75633, and UniProt
      E0J145)

<400> 1
atgtcgagtt taacctgctt taaagcctat gatattcgcg ggaaattagg cgaagaactg      60

aatgaagata tcgcctggcg tattgggcgt gcctatggcg aatttctcaa accgaaaacc     120

attgtgttag gcggtgacgt ccgcctcacc agcgaaacct taaaactggc gctggcgaga     180

ggtttacagg atgcgggcgt cgatgtgctg gatatcggca tgtccggcac cgaagagatc     240

tatttcgcca cgttccatct cggcgtggat ggcggcatcg aagttaccgc cagccataat     300

ccgatggatt ataacggcat gaagctggtt cgcgaggggg ctcgcccgat cagcggagat     360

accggactgc gcgacgtcca gcgtctggct gaagccaacg actttcctcc cgtcgatgaa     420

accaaacgcg gtcgctatca gcaaatcaac ctgcgtgacg cttacgttga tcacctgttc     480

ggttatatca atgtcaaaaa cctcacgccg ctcaagctgg tgatcaactc cgggaacggc     540

gcagcgggtc cggtggtgga cgctatcgaa gcccgcttta aagccctcgg cgcacctgtg     600

gaattgatca aagtgcacaa cacgccggac ggcaatttcc ccaacggtat tcctaacccg     660

ctgctgccgg aatgtcgcga cgacacccgc aatgcggtca tcaaacacgg cgcggatatg     720

ggcattgcct ttgatggcga ttttgaccgc tgtttcctgt ttgacgaaaa agggcagttt     780

atcgagggct actacattgt cggcctgctg gcagaagcgt tcctcgaaaa aaatcccggc     840

gcgaagatca tccacgatcc acgtctctcc tggaataccg ttgatgtggt gaccgccgcg     900

ggcggcacac cggtgatgtc gaaaaccgga cacgccttta ttaaagaacg tatgcgcaag     960

gaagacgcca tctacggtgg cgaaatgagc gcccaccact atttccgtga tttcgcttac    1020

tgcgacagcg gcatgatccc gtggctgctg gtcgccgaac tggtgtgtct gaaaggaaaa    1080

acgctgggcg aactggtgcg cgaccggatg gcagcgtttc cggcaagcgg tgagatcaac    1140

agcaaactgg cgcaacccgt tgaggcgatt aatcgcgtcg aacagcattt tagccgcgag    1200

gcgctggcgg tggatcgcac cgatggcatc agcatgacct ttgccgactg gcgctttaac 1260

ctgcgctcct ccaacaccga accggtggtg cggttgaatg tggaatcgcg cggtgatgtg 1320

ccgctgatgg aagaaaagac aaaacttatc cttgcgttat tgaacaagta a 1371

<210> 2
<211> 1440
<212> DNA
<213> Bacteria <prokaryote>

<220>
<223> ManC (corresponding to NCBI-ProteinID ADT75634, and UniProt
      E8PZ98)

<400> 2
atgaacaata ataaaattat tacacctatc attatggcag gtggttcagg cagtcggttg 60

tggcgactat caagaattct ctatccgaaa caatttctta gcctaaacgg tagtcatacc 120

atgcttcaaa caacggctaa tcgtctggat ggtttggatt gtaccaaccc ttatgtcatt 180

tgtaatgaac aacaccgctt tatagttgct gaacagctta gaaaaatcga tagattgact 240

tcaaagaata tcatccttga gcctgttggg cgtaacactg cccctgcaat tgcattagcg 300

gcgttgctga tgtctaagtc tgataaaagt gcagatgatc ttatgctcgt actggctgca 360

gatcacgtta tattagatga agaaaaattt tgtaacgctg ttagatcggc aattccatac 420

gctgctgatg ggaaattggt aacatttggt ataattccag acaaagcaga aactggttat 480

ggttatatac atcgaggaca atatattaat caggaagatt cggatgcatt tatagtgtca 540

tcatttgttg aaaagccaaa tcatgagaca gccactaaat atcttgcttc cggtgagtat 600

tattggaata gcggtatgtt tttgtttagt gcaaatcgtt atatagagga acttaaacaa 660

tttcggcctg atattttatc cgcttgtgaa aaagcaattg cttcagcgaa ctttgacctt 720

gattttgtgc gtttagatga aagttctttc tctaagtgtc ctgaagagtc aattgattac 780

gctgtaatgg aaaaaacaaa agacgcaatt gttattccaa tggatgctgg ctggagtgat 840

gtcggttcat ggtcttctct ttgggaaatt aatgataaag actcagacgg caacgtaata 900

gttggggata ttttctctca tgaaacaaag aattctttca tatatgccga atcgggaatt 960

gttgctacag tgggagtgga aaatttagtt gttgtccaaa caaaggatgc cgttcttgtc 1020

tcagagagaa ataaagttca ggatgtaaag aaaatagtcg aacaaattaa aaattcaggt 1080

cgtagcgagc attatgttca tcgcgaagta tatcgtcctt ggggtaaata tgattccatt 1140

gacacagggg agcgttatca ggtcaaacgt ataacagtaa atcctggtga aggactttct 1200

ttacaaatgc accatcatag ggcagaacat tggatcatag tttctggaac tgcaaaggtg 1260

actataggtt ctgaaactaa gattcttagc gaaaatgaat ctgtttacat acctcttggt 1320

gtaatacact gcttggaaaa tccagggaaa attcctcttg atttaattga agttcgttct 1380

ggatcttatt tagaagaaga cgatgttatc cgttttcagg accgatatgg tcgcagctaa 1440

<210> 3
<211> 1122
<212> DNA
<213> Bacteria <prokaryote>

<220>
<223> GMD from E. coli (GMD_EC) (corresponding to NCBI-ProteinID
      ADT75653, and UniProt E0J125)

<400> 3
atgtcaaaag tcgctctcat caccggtgta accggacaag acggttctta cctggcagag 60

tttctgctgg aaaaaggtta cgaggtgcat ggtattaagc gtcgtgcatc gtccttcaac 120

accgagcgcg tggatcacat ttatcaggat ccgcacacct gcaacccgaa attccatctg 180

cattatggcg acctgagtga tacctccaac ctgacgcgca ttttgcgtga agtgcagccg 240

gatgaagtgt acaacctggg cgcaatgagt cacgttgcgg tttcttttga gtcaccggaa 300

tataccgcag acgtcgatgc gatgggtacg ctgcgcctgc tggaggcgat ccgcttcctc 360

ggtctggaaa agaaaacccg tttctatcag gcttccacct ctgaactgta cggtctggtg 420

caggaaattc cgcagaaaga aaccacgccg ttctacccgc gatctccgta tgcggtcgcc 480

aaactgtatg cctactggat caccgttaac taccgcgaat cctacggcat gtacgcctgt 540

aacggtattc tcttcaacca tgaatccccg cgccgcggcg aaaccttcgt tacccgcaaa 600

atcacccgcg caatcgccaa catcgcccag gggctggagt cgtgcctgta cctcggcaat 660

atggattccc tgcgtgactg gggccacgcc aaagactacg taaaaatgca gtggatgatg 720

ctgcaacagg aacagccgga agatttcgtt atcgcgaccg gcgttcagta ctccgtgcgt 780

cagttcgtgg aaatggcagc ggcacagctg ggcatcaaac tgcgctttga aggcacgggt 840

gttgaagaga agggcattgt ggtttccgtc accgggcatg acgcgccggg cgttaaaccg 900

ggtgatgtga ttatcgccgt tgatccgcgt tacttccgtc cggctgaagt tgaaacgctg 960

ctcggcgacc cgaccaaagc gcacgaaaaa ctgggctgga aaccggaaat caccctcaga 1020

gagatggtgt ctgaaatggt ggctaatgac ctcgaagcgg cgaaaaaaca ctctctgctg 1080

aaatctcacg gctacgacgt ggcgatcgcg ctggagtcat aa 1122

<210> 4
<211> 966
<212> DNA
<213> Bacteria <prokaryote>

<220>
<223> WcaG from E. coli (WcaG_EC) (corresponding to NCBI-ProteinID

ADT75652, and UniProt E0J126)

<400> 4
atgagtaaac aacgcatttt tatcgctggc catcgtggga tggtcggttc cgccatcacg      60

cggcagctcg aacagcgcgg tgatgtggaa ctggtattac gcacccgcga cgagctgaac     120

ctgctggaca gccgcgcggt gcatgatttc tttgccagcg aacgcattga ccaggtctat     180

ctggcggcgg cgaaagtggg cggcattgtt gccaacaaca cctatccggc ggatttcatc     240

taccagaaca tgatgattga gagcaacatc attcacgccg cgcatcagaa cgacgtgaac     300

aaactgctgt ttcttggatc gtcctgtatc tacccgaaac tggcaaaaca gccgatggca     360

gaaagcgagt tattgcaggg cacgctggag ccgaccaacg agccttacgc cattgccaaa     420

atcgccggga tcaaactgtg cgaatcatac aaccgccagt acggacgcga ttaccgctca     480

gtcatgccga ccaacctgta tggcccgcat gacaacttcc acccgagtaa ttcgcatgtg     540

atccctgcat tgctgcgtcg cttccacgag gcgacggcac agaatgcacc ggacgtggtg     600

gtatggggca gcggtacacc gatgcgtgaa ttcctgcacg tcgatgatat ggcggcggcg     660

agcattcatg tcatggagct ggcgcacgaa gtctggctgg agaacaccca gccgatgctg     720

tcgcacatta acgtcggcac gggcgttgac tgcactatcc gcgagctggc gcaaaccatc     780

gccaaagtgg tgggttacaa aggtcgggtg gtttttgatg ccagcaaacc ggatggtacg     840

ccgcgcaaac tgcttgatgt gacgcgcctg catcagcttg gctggtatca cgaaatctca     900

ctggaagcgg ggcttgccag cacttaccag tggttccttg agaatcaaga ccgctttcgg     960

gggtaa                                                                966


<210> 5
<211> 1083
<212> DNA
<213> Bacteria <prokaryote>


<220>
<223> Gmd from Bacteroides fragilis (Gmd_BF)

<400> 5
atgattgaca gaatggataa aaacgcaaaa atttatgtag ccggacacca cggactggtg      60

ggttcggcta tatggaaaaa tctgcaggaa aaggggtata cgaatctggt gggacgcaca     120

cataaggaac tggacttatt ggacggtgcg accgtaaagc agttttttga tgaggaaatg     180

ccggagtacg tgtttttggc tgccgctttt gtcggaggaa tcatggccaa tagtatctac     240

cgtgcggact ttatctataa gaacttacag atacagcaga acgtgatcgg agaaagtttc     300

cggcatcagg tgaaaaaact gctctttctg ggcagtacct gcatctatcc gagggatgcc     360

gaacagccga tgaaagagga cgtcttgctc acttctccac tggaatatac caatgaacct     420

tatgccatag ccaaaatagc cggactgaaa atgtgcgaaa gcttcaacct gcaatatgga     480

acgaactaca tcgccgtgat gccgaccaac ctgtatgggc cgaacgataa ctttgacttg 540

gaacgcagtc atgtgctgcc tgccatgatc cgtaaagttc acttggcaca ctgcctgaaa 600

aaaggagatt gggaggccgt gcgtaaagat atgaacctgc gtccggtaga aggcatcagt 660

ggtgccaact ccaacgaaga gatcctccgg attctccgga aatacggcat tactgaaacg 720

gaggtgacac tttggggaac aggaacgcct ctaagggaat ttctttggag tgaagaaatg 780

gcagatgcca gtgtcttcgt aatggaacat gtggacttca aggataccta caaaaccggt 840

gcaaaagaca tccgcaactg ccacatcaat ataggtaccg gcaaagaaat tacaatccgc 900

gaactggccg gactgattgt aaatacagtc ggctatcagg gtgaactgac ttttgacagc 960

agtaaaccgg acggaaccat gcgaaaactt accgatccgt cgaaattgca caacctcgga 1020

tggcatcata agatcgatat tgaagagggg gtacagaaaa tgtacgagtg gtatctggga 1080

taa 1083

<210> 6
<211> 1083
<212> DNA
<213> Bacteria <prokaryote>

<220>
<223> WcaG from Bacteroides fragilis (WcaG_BF)

<400> 6
atgattgaca gaatggataa aaacgcaaaa atttatgtag ccggacacca cggactggtg 60

ggttcggcta tatggaaaaa tctgcaggaa aaggggtata cgaatctggt gggacgcaca 120

cataaggaac tggacttatt ggacggtgcg accgtaaagc agttttttga tgaggaaatg 180

ccggagtacg tgtttttggc tgccgctttt gtcggaggaa tcatggccaa tagtatctac 240

cgtgcggact ttatctataa gaacttacag atacagcaga acgtgatcgg agaaagtttc 300

cggcatcagg tgaaaaaact gctctttctg ggcagtacct gcatctatcc gagggatgcc 360

gaacagccga tgaaagagga cgtcttgctc acttctccac tggaatatac caatgaacct 420

tatgccatag ccaaaatagc cggactgaaa atgtgcgaaa gcttcaacct gcaatatgga 480

acgaactaca tcgccgtgat gccgaccaac ctgtatgggc cgaacgataa ctttgacttg 540

gaacgcagtc atgtgctgcc tgccatgatc cgtaaagttc acttggcaca ctgcctgaaa 600

aaaggagatt gggaggccgt gcgtaaagat atgaacctgc gtccggtaga aggcatcagt 660

ggtgccaact ccaacgaaga gatcctccgg attctccgga aatacggcat tactgaaacg 720

gaggtgacac tttggggaac aggaacgcct ctaagggaat ttctttggag tgaagaaatg 780

gcagatgcca gtgtcttcgt aatggaacat gtggacttca aggataccta caaaaccggt 840

gcaaaagaca tccgcaactg ccacatcaat ataggtaccg gcaaagaaat tacaatccgc 900

gaactggccg gactgattgt aaatacagtc ggctatcagg gtgaactgac ttttgacagc 960

agtaaaccgg acggaaccat gcgaaaactt accgatccgt cgaaattgca caacctcgga 1020

tggcatcata agatcgatat tgaagagggg gtacagaaaa tgtacgagtg gtatctggga 1080

taa 1083

<210> 7
<211> 900
<212> DNA
<213> Bacteria <prokaryote>

<220>
<223> FucT from H. pylori (HpFucT)

<400> 7
atggctttta aggtggtgca aatttgcggg gggcttggga atcaaatgtt tcaatacgct 60

ttcgctaaaa gtttgcaaaa acactctaat acgcctgtgc tgttagatat tacttctttt 120

gattggagta atagaaaaat gcaattagaa cttttcccta ttgatttgcc ctatgcgagt 180

gaaaaagaaa tcgctatagc taaaatgcaa cacctcccca agctagtaag aaatgtgctc 240

aaatgcatgg ggtttgatag ggtgagtcaa gaaatcgttt ttgaatacga gcctaaattg 300

ttaaagacaa gccgcttgac ttatttttat ggctattttc aagatccacg atattttgat 360

gctatatccc ctttaatcaa gcaaaccttc accctacccc ccccccccga aaatggaaat 420

aataaaaaaa aagaggaaga ataccaccgc aagcttgctt tgattttagc cgctaaaaac 480

agcgtgtttg tgcatataag aagaggggat tatgtgggga ttggctgtca gcttggtatt 540

gattatcaaa aaaaggcgct tgagtatatg gcaaagcgcg tgccaaacat ggagcttttt 600

gtgttttgcg aagacttaga attcacgcaa aatcttgatc ttggctaccc ttttatggac 660

atgaccacta gggataaaga agaagaagcg tattgggata tgctgctcat gcaatcttgc 720

aagcatggca ttatcgctaa cagcacttat agctggtggg cggcctattt gataaacaat 780

ccagaaaaaa tcattattgg ccccaaacac tggctttttg ggcatgagaa tatcctttgt 840

aaggaatggg tgaaaataga atcccatttt gaggtgaaat cccaaaagta taacgcttaa 900

<210> 8
<211> 873
<212> DNA
<213> Bacteria <prokaryote>

<220>
<223> Codon optimized FucT from H. mustelae (codon optimized HmFucT)

<400> 8
atggcacagg tcgtggactt caagatcgtc caggtccacg gcggcctcgg caaccagatg 60

ttccagtacg cattcgcaaa gagcctccag acccacctca acatcccggt cctcctcgac 120

accacctggt tcgactacgg caaccgtgag ctcggcctcc acctcttccc gatcgacctc 180

cagtgcgcaa gcgcacagca gatcgcagca gcacacatgc agaacctccc gcgtctcgtc 240

cgtggcgcac tccgtcgtat gggcctcggc cgtgtcagca aggagatcgt cttcgagtac 300

atgccggagc tcttcgagcc gagccgtatc gcatacttcc acggctactt ccaggacccg 360

cgttacttcg aggacatcag cccgctcatc aagcagacct tcaccctccc gcacccgacc 420

gagcacgcag agcagtacag ccgtaagctc agccagatcc tcgcagcaaa gaacagcgtc 480

ttcgtccaca tccgtcgtgg cgactacatg cgtctcggct ggcagctcga catcagctac 540

cagctccgtg caatcgcata catggcaaag cgtgtccaga acctcgagct cttcctcttc 600

tgcgaggacc tcgagttcgt ccagaacctc gacctcggct acccgttcgt cgacatgacc 660

acccgtgacg gcgcagcaca ctgggacatg atgctcatgc agagctgcaa gcacggcatc 720

atcaccaaca gcacctacag ctggtgggca gcatacctca tcaagaaccc ggagaagatc 780

atcatcggcc cgagccactg gatctacggc aacgagaaca tcctctgcaa ggactgggtc 840

aagatcgaga gccagttcga gaccaagagc taa 873

<210> 9
<211> 1254
<212> DNA
<213> Bacteria <prokaryote>

<220>
<223> LacY from E. coli (LacY_EC)

<400> 9
atgtactatt taaaaaacac aaacttttgg atgttcggtt tattcttttt cttttacttt 60

tttatcatgg gagcctactt cccgtttttc ccgatttggc tacatgacat caaccatatc 120

agcaaaagtg atacgggtat tatttttgcc gctatttctc tgttctcgct attattccaa 180

ccgctgtttg gtctgctttc tgacaaactc gggctgcgca aatacctgct gtggattatt 240

accggcatgt tagtgatgtt tgcgccgttc tttattttta tcttcgggcc actgttacaa 300

tacaacattt tagtaggatc gattgttggt ggtatttatc taggcttttg ttttaacgcc 360

ggtgcgccag cagtagaggc atttattgag aaagtcagcc gtcgcagtaa tttcgaattt 420

ggtcgcgcgc ggatgtttgg ctgtgttggc tgggcgctgt gtgcctcgat tgtcggcatc 480

atgttcacca tcaataatca gtttgttttc tggctgggtt ctggctgtgc actcatcctc 540

gccgttttac tctttttcgc caaaacggat gcgccctctt ccgccacggt tgccaatgcg 600

gtaggtgcca accattcggc atttagcctt aagctggcgc tggaactgtt cagacagcca 660

aaactgtggt ttttgtcact gtatgttatt ggcgtttcct gcacctacga tgtttttgac 720

caacagtttg ctaatttctt tacttcgttc tttgctaccg gtgaacaggg tacgcgggta 780

tttggctacg taacgacaat gggcgaatta cttaacgcct cgattatgtt ctttgcgcca 840

ctgatcatta atcgcatcgg tgggaaaaac gccctgctgc tggctggcac tattatgtct 900

gtacgtatta ttggctcatc gttcgccacc tcagcgctgg aagtggttat tctgaaaacg 960

ctgcatatgt ttgaagtacc gttcctgctg gtgggctgct ttaaatatat taccagccag 1020

tttgaagtgc gtttttcagc gacgatttat ctggtctgtt tctgcttctt taagcaactg 1080

gcgatgattt ttatgtctgt actggcgggc aatatgtatg aaagcatcgg tttccagggc 1140

gcttatctgg tgctgggtct ggtggcgctg ggcttcacct taatttccgt gttcacgctt 1200

agcggccccg gcccgctttc tctactgcgt cgtcaggtga atgaagtcgc ttaa 1254

<210> 10
<211> 1884
<212> DNA
<213> Bacteria <prokaryote>

<220>
<223> LacY from Lactobacillus delbrueckii (LacY_Ldb)

<400> 10
atgaagaaaa agcttgtctc acgcttgtcg tacgcggccg gtgcctttgg caacgacgtc 60

ttctacgcga ctctgtcaac ctactttatc gtcttcgtca ccacccacct ctttaatgcc 120

ggtgaccaca agatgatctt tatcatcacc aacttgatca ccgccatccg gatcggggaa 180

gtcctgctcg accccttgat cggtaacgcc atcgaccgga ccgaaagccg gtgggggaag 240

ttcaagccct gggttgtggg cggggggatc atcagctcat tagccctctt agccctcttt 300

accgactttg gcggcattaa ccaaagcaaa cccgttgttt acttagtaat cttcggtatt 360

gtttacttga ttatggatat cttctactca tttaaagaca ctggcttctg ggccatgatc 420

ccggccttgt ccctggattc ccgggaaaga gagaagacct ccaccttcgc cagagtcggc 480

tccaccatcg gggccaacct ggtcggggta gtcatcaccc caatcatcct cttcttctcg 540

gctagcaagg ccaaccccaa cggggataag cagggctggt tcttctttgc cttgatcgtg 600

gccattgtcg gcatcttgac ctcaattacc gttggtcttg gtactcacga agtaaaatcc 660

gccctgcggg aaagcaatga aaagaccact ttgaagcagg tctttaaggt cctggggcaa 720

aacgaccagc tcctctggct ggcctttgcc tactggtttt acggcctggg tatcaacacc 780

ctgaacgctc tgcaacttta ctacttctca tacatcttag gcgatgcccg cggctacagc 840

ctgctttaca ccatcaacac ctttgtcggt ttaatctctg catccttctt cccatcactg 900

gccaagaagt tcaacagaaa tcgcctcttc tacgcctgca tcgcggtgat gctgttaggg 960

atcggggtct tctccgtggc cagcggttct ctggccctgt cccttgttgg ggcagaattc 1020

ttctttattc cgcagcctct ggccttcctg gtcgttttga tgatcatctc tgacgctgtt 1080

gaatacggcc agctgaaaac tggccacaga gacgaagctt tgaccctgtc tgtccggcca 1140

ttggtcgata agctgggcgg ggccttgtcc aactggtttg tttccttgat tgccttaact 1200

gccggcatga ccactggggc gactgcctca acaattacag ctcatggcca gatggtcttc 1260

aagttagcta tgtttgcctt accggcagtc atgctcttga tcgctgtttc tattttcgcc 1320

aaaaaggtct tcttgactga agaaaagcac gcggaaatcg tcgaccagct ggaaactcaa 1380

ttcggccaaa gccatgccca aaagccggcg caagctgaaa gcttcacttt ggccagccca 1440

gtctccggac aattaatgaa cctggacatg gttgacgacc cggtctttgc cgacaaaaag 1500

ttaggcgacg gctttgccct ggtgccagca gacggtaagg tctacgcgcc atttgccggt 1560

actgtccgcc agctggccaa gacccggcac tcgatcgtcc tggaaaatga acatggggtc 1620

ttggtcttga ttcaccttgg cctgggcacg gccaaattaa acgggactgg ctttgtcagc 1680

tatgttgaag agggcagcca ggtagaagcc ggccagcaga tcctggaatt ctgggacccg 1740

gcgatcaagc aggccaagct ggacgacacg gtaatcgtga ccgtcatcaa cagcgaaact 1800

ttcgcaaata gccagatgct cttgccgatc ggccacagcg tccaagccct ggatgatgta 1860

ttcaagttag aagggaagaa ttag 1884

<210> 11
<211> 1092
<212> DNA
<213> Bacteria <prokaryote>

<220>
<223> LacI from Escherichia Coli

<400> 11
tcactgcccg ctttccagtc gggaaacctg tcgtgccagc tgcattaatg aatcggccaa 60

cgcgcgggga gaggcggttt gcgtattggg cgccagggtg gtttttcttt tcaccagtga 120

gacgggcaac agctgattgc ccttcaccgc ctggccctga gagagttgca gcaagcggtc 180

cacgctggtt tgccccagca ggcgaaaatc ctgtttgatg gtggttaacg gcgggatata 240

acacgagctg tcttcggtat cgtcgtatcc cactaccgag atatccgcac caacgcgcag 300

cccggactcg gtaatggcgc gcattgcgcc cagcgccatc tgatcgttgg caaccagcat 360

cgcagtggga acgatgccct cattcagcat ttgcatggtt tgttgaaaac cggacatggc 420

actccagtcg ccttcccgtt ccgctatcgg ctgaatttga ttgcgagtga gatatttatg 480

ccagcccgcc agacgcagac gcgccgagac agaacttaat gggcccgcta acagcgcgat 540

ttgctggtga cccaatgcga ccagatgctc cacgcccagt cgcgtaccgt cttcatggga 600

gaaaataata ctgttgatgg gagtctggtc agagacatca agaaataaag caggaacatt 660

agcgcaggct gcttccacag caatggcatc ctggtcatcc agcggatagt taatgatcag 720

cccactgacg cgttgcgcga gaaggttgtg caccgccgct ttacaggctt cgacgccgct 780

tcgttctacc atcgacacca ccacgctggc acccagttga tcggcgcgag atttaatcgc 840

cgcgacaatt tgcgacggcg cgtgcagggc cagactggag gtggcaacgc caatcagcaa 900

cgactgtttg cccgccagtt gttgtgccac gcggttggga atgtaattca gctccgccat 960

cgccgcttcc actttttccc gcgttttcgc agaaacgtgg ctggcctggt tcaccacgcg 1020

ggaaacggtc tgataagaga caccggcata ctctgcgaca tcgtatagcg ttactggttt 1080

cacattcacc ac 1092